# EUROPÄISCHE PATENTSCHRIFT

(11) **EP 2 799 835 B1**
(45) Veröffentlichungstag und Bekanntmachung des Hinweises auf die Patenterteilung: **18.12.2019**
(21) Anmeldenummer: 14161157.4
(22) Anmeldetag: 21.03.2014
(51) Int. Cl.: G01N 1/12

(54) **Probennehmer und Verfahren zur Probenentnahme**
Sampler and method for sampling
Dispositif de prélèvement d'échantillon et procédé de prélèvement d'échantillon

(30) Priorität: 30.04.2013 DE 102013207959; 29.11.2013 DE 102013224565
(43) Veröffentlichungstag der Anmeldung: 05.11.2014
(60) Teilanmeldung: 19199871.5
(73) Patentinhaber: Heraeus Electro-Nite International N.V., 3530 Houthalen (BE)
(72) Erfinder: Cappa, Guido, 3530 Houthalen (BE); Broekmans, Gerrit, 3583 Paal (BE)
(74) Vertreter: Heraeus IP

(56) Entgegenhaltungen:
- EP-A1- 0 556 474
- EP-A2- 2 525 207
- WO-A2-2009/030206
- GB-A- 1 528 922
- US-A- 3 460 393

## Beschreibung

Die Erfindung betrifft einen Probennehmer nach Anspruch 1, mit einer Probenkammer für eine sich aus einer Schmelze bildende Probe, vorzugsweise für eine sich bildende Probe aus einer Metallschmelze, insbesondere aus einer Roheisen- oder Stahlschmelze.

Weiteren betrifft die Erfindung ein Verfahren nach Anspruch 7, zur Probenentnahme aus einer Schmelze mit einer Schmelztemperatur von größer als 600 °C, insbesondere einer Metallschmelze, vorzugsweise einer Roheisen- oder Stahlschmelze.

Nach dem heute bekannten Stand der Technik ist es möglich, aus einer Schmelze, beispielsweise einer Metallschmelze, Proben zu entnehmen.

Aus der EP 2 397 834 A2 ist beispielsweise eine Messsonde zur Messung und Probennahme in einer Metallschmelze mit einem an einer Lanze angeordneten Messkopf bekannt, wobei der Messkopf mindestens einen Temperatursensor und eine Probenkammer trägt, wobei die Probenkammer zumindest teilweise von dem Messkopf umgeben ist und einen durch den Messkopf hindurch verlaufenden Einfüllkanal aufweist. Das Einfüllrohr ist beispielsweise ein Quarzglasrohr.

Aus der US 3,646,816 A ist ein Probenaufnehmer bekannt, bei dem die Probe durch Eintauchen in ein Schmelzbad entsteht. Dabei werden mittels unterschiedlich ausgeformter Probenkammern zum einen eine flache Probe und zum anderen eine nadelförmige Probe erzeugt, wobei mittels eines Aluminiumrohres im Bereich des Eingangsbereiches des Schmelzeneintrittsrohres eine Deoxidation verhindert wird. Mittels Öffnungen wird die bei der Probenaufnahme erzeugte komprimierte Luft aus dem Probennehmer abgelassen. Mittels Metallscheiben im Bereich der Probenkammern werden dabei die Proben heruntergekühlt.

Des Weiteren ist in der DE 32 00 010 A1 offenbart, dass eine Lanze zur Entnahme von metallischen Tauchproben für die spektralanalytische Untersuchung verwendet wird, wobei der in die metallische Schmelze eintauchende Endabschnitt der Lanze eine Tauchkokille mit einem geschlossenen Eintrittskanal aufweist, wobei die Tauchkokille in einer Schutzgasatmosphäre angeordnet ist und die durch den Eintrittskanal aufsteigende Probenmenge das Schutzgas komprimiert und/oder verdrängt. Dabei weist die Lanze in einer Ausführung ein Überdruckventil und in einer Ausführung ein Ventil zum Spülen der Tauchkokille mit Inertgas und zum gasdichten Verschließen auf.

Ferner ist aus der DE 10 2011 121 183 A1 bekannt, dass bei einem Probennehmer ein Kühlkörper aus gut wärmeleitendem Kupfer verwendet wird, so dass ein schneller Wärmeabfluss aus der in die Kühlkammer eingelaufenen Probe erfolgt und diese folglich schnell abgekühlt wird, wobei der Kühlkörper aus zwei Körpern besteht, die die innere Wand der Probenkammeranordnung bilden. Ferner ist aus der Schrift bekannt, dass bei Entnahme der Probe aus der Probenkammer diese von einem Inertgas umgeben ist.

Nachteilig bei dem heutzutage bekannten Stand der Technik ist, dass die in der Probenkammer aufgenommene Schmelze, die später die Probe selbst bildet, nur sehr langsam in der Probenkammer abkühlt. Nachfolgende Messungen an der abgekühlten Probe sind daher nur zeitlich vergert möglich. Ferner treten beispielsweise Oxidationsreaktionen aufgrund Umgebungsluft an der noch nicht abgekühlten Probe auf, wenn diese im heißen Zustand aus dem Probennehmer entfernt wird.

Eine Aufgabe der Erfindung ist es daher, eine Möglichkeit zu schaffen, mittels der oben beschriebene Nachteile verringert oder vermieden werden können. Insbesondere soll eine Möglichkeit geschaffen werden, eine in einer Probenkammer aufgenommene Schmelze schnell und einfach zu kühlen, so dass die während der Kühlung entstehende feste Probe aus der Schmelze zeitnah nach der Aufnahme der Schmelze entnommen werden kann. Ferner sollen Reaktionen der Probe mit beispielsweise Umgebungsluft durch eine schnelle Kühlung vermieden werden.

Eine weitere Aufgabe ist es, eine Möglichkeit zu schaffen, die Probenkammer mit Hilfe von technisch einfachen und kostengünstigen Mitteln mit einer Schmelze füllen zu können.

Eine weitere Aufgabe ist es, ein Verfahren zur Probenentnehme aus einer Schmelze zu schaffen, mittels dem die durch die Schmelze gebildete Probe technisch einfach und zeitlich schnell heruntergekühlt werden kann, um diese dann beispielsweise analytisch zu untersuchen.

Eine weitere Aufgabe ist es, ein Verfahren zur Probenentnahme aus einer Schmelze zu schaffen, mittels der die Probenkammer schnell füllbar ist.

Die erstgenannte Aufgabe der Erfindung wird dadurch gelöst, dass der Probennehmer zwischen einem Bereich der Außenseite des inneren Kühlkörpers und dem diesem Bereich der Außenfläche des inneren Kühlkörpers gegenüber liegenden Bereich der Außenfläche des oberen Kühlkörpers zumindest einen Spalt für die Durchleitung von zumindest einem Gas, vorzugsweise von einem Inertgas, insbesondere Argon oder Stickstoff, aufweist und dass das Volumen des jeweiligen Kühlkörpers größer als das Volumen des Spaltes ist, vorzugsweise ein Verhältnis von zumindest 3:1, insbesondere zumindest 5:1, vorzugsweise zumindest 10:1, insbesondere zumindest 20:1, bildet, so dass eine bessere Kühlleistung des Probennehmers entsteht.

Die nachfolgende Aufgabe wird zum Beispiel dadurch gelöst, dass in dem Probenhalter ein mit der Zuleitung und der Ableitung einerseits und mit der Gasleitung andererseits verbundener Schalter angeordnet ist, mit dem entweder die Zuleitung oder die Ableitung mit der Gasleitung verbindbar sind.

Diese Aufgabe wird zum Beispiel auch dadurch gelöst, dass mit einem Ende des Lanzenkörpers ein Probenhalter verbindbar ist, mit dem ein Probennehmer nach einem der Ansprüche 1 bis 6 verbindbar ist, wobei die Vorrichtung zumindest eine Zuleitung zur Einleitung von Gas durch das Kontaktstück hindurch in den Probennehmer und zumindest eine Ableitung zum Absaugen von Gas durch das Kontaktstück hindurch aus dem Probennehmer und zumindest eine in dem Probennehmer verlaufende und mit der Probenkammer verbundene Gasleitung aufweist.

Die weitere Aufgabe bezüglich des erstgenannten Verfahrens der Erfindung wird dadurch gelöst, dass vor dem Eintauchen zumindest ein Gas, vorzugsweise ein Inertgas, insbesondere Argon oder Stickstoff, in den Probennehmer zugeführt wird, wobei das Gas durch zumindest ein Einfüllstück, vorzugsweise ein Einfüllrohr, aus dem Probennehmer wieder ausströmt, anschließend der Probennehmer in die Schmelze eingetaucht wird, danach die Zufuhr von Gas geändert wird, insbesondere unterbrochen oder in ihrer Richtung umgekehrt wird, nachfolgend sich die Probenkammer mit Schmelze füllt, nachfolgend während oder nach dem Füllen der Probenkammer mit Schmelze erneut Gas in den Probennehmer zugeführt wird, so dass zumindest die Probenkammer durch das zugeführte Gas gekühlt wird.

Die zuletzt genannte Aufgabe bezüglich des Verfahrens zur Probenentnahme wird dadurch gelöst, dass vor dem Eintauchen zumindest ein Gas, vorzugsweise ein Inertgas, insbesondere Argon oder Stickstoff, in den Probennehmer durch zumindest eine Zuleitung und zumindest eine Gasleitung zugeführt wird, wobei das Gas durch zumindest ein Einfüllstück, vorzugsweise ein Einfüllrohr, aus dem Probennehmer wieder ausströmt, anschließend der Probennehmer in die Schmelze eingetaucht wird, danach die Zufuhr von Gas geändert wird, insbesondere unterbrochen oder in ihrer Richtung umgekehrt wird, indem ein Schalter in den Probenhalter von der Stellung A in die Stellung B umgeschaltet wird, nachfolgend sich die Probenkammer mit Schmelze füllt, nachfolgend während oder nach dem Füllen der Probenkammer mit Schmelze erneut Gas in den Probennehmer zugeführt wird, indem der Schalter von Stellung B in Stellung C umgeschaltet wird und wobei zumindest die Probenkammer durch das zugeführte Gas gekühlt wird.

Die Erfindung betrifft somit als Hauptanspruch und als Nebenansprüche zumindest einen Probennehmer gemäß Anspruch 1 und Verfahren zur Probenentnahme gemäß Anspruch 7.

Ein Ausführungsbeispiel zeigt eine Vorrichtung zur Herstellung eines Probennehmers nach einem der Ansprüche 1 bis 6, wobei die Vorrichtung geeignete Mittel zur Herstellung des Probennehmers nach einem der Ansprüche 1 bis 6 aufweist.

Ein weiteres Ausführungsbeispiel zeigt ein Verfahren zur Herstellung eines Probennehmers nach einem der Ansprüche 1 bis 6 mit Hilfe einer Vorrichtung gemäß der vorhergehenden Ausführung.

In einem Ausführungsbeispiel handelt es sich um eine Vorrichtung zur Durchführung von Probenentnahmen in Metallschmelzen mit einer Lanze, insbesondere in Stahlschmelze mit einer Sublanze, wobei die Lanze einen Lanzenkörper aufweist, wobei an einem Ende des Lanzenkörpers ein Probenhalter anordenbar ist mit einem Kontaktstück zur Aufnahme eines Probennehmers gemäß der Erfindung, wobei die Vorrichtung zumindest eine Zuleitung zur Einleitung von Gas durch das Kontaktstück hindurch in den Probennehmer und zumindest eine Ableitung zum Absaugen von Gas durch das Kontaktstück hindurch aus dem Probennehmer und zumindest eine durch das Kontaktstück hindurch verlaufende und durch das Kontaktstück hindurch verlaufende und mit der Probenkammer verbundene Gasleitung aufweist, wobei der Schalter statt in den Probenhalter in dem Lanzenkörper angeordnet ist.

Ein weiteres Ausführungsbeispiel zeigt eine Vorrichtung zur Herstellung eines Probenhalters wobei die Vorrichtung geeignete Mittel zur Herstellung des Probenhalters aufweist.

Ein weiteres Ausführungsbeispiel zeigt ein Verfahren zur Herstellung eines Probenhalters mit Hilfe einer Vorrichtung gemäß vorhergehender Ausführung.

In den Unteransprüchen sind jeweils bevorzugte Ausführungsformen gemäß der Erfindung beansprucht.

Eine Änderung umfasst im Sinne der Erfindung sowohl die Reduzierung, das Ausschalten oder die Umkehr. Bei einer Umkehr, das heißt der Umkehr der Richtung, der Zufuhr wird ein Unterdruck in dem Probennehmer und somit auch in der Probenkammer erzeugt. Diese drei Zustände der Änderung können auch in zeitlich folgender Kombination auftreten.

Mittels des Probennehmers gemäß der Erfindung ist es möglich, eine Probe, die aus einer Schmelze gebildet worden ist, mit Hilfe der Kühlkörper technisch einfach und auch schnell auf eine Temperatur herunter zu kühlen, bei der die Probe aus der Probenkammer entnommen werden kann oder alternativ in dem Probennehmer weiter verwendet werden kann. Eine Entnahme in Form eines Entfernens ist durch Fallenlassen oder Manipulierung des Probennehmers möglich, so dass die Probe bei Zerstörung des Probennehmers freigelegt wird. Des Weiteren ist mittels des Probennehmers eine kostengünstige Kühlung der aus der Schmelze sich bildende Probe möglich. Auch mittels des erfindungsgemäßen Verfahrens zur Probenentnahme aus einer Schmelze kann eine Probe mit Hilfe des erneut in den Probennehmer zugeführten Gases einfach, kostengünstig und schnell gekühlt werden. Eine gekühlt Probe reagiert nicht mehr mit beispielsweise Umgebungsluft oder alternativ ist eine auftretende Reaktion oder Veränderung der Probe durch die Kühlung reduziert. Ferner ist es weiter vorteilhaft möglich, dass der Probennehmer nicht für eine Analyse der erstarrten Probe aufgrund seiner Abmessung und der Verwendung eines Inertgases, beispielsweise Argon, in dem Probennehmer präpariert werden muss. Somit sind keine zusätzlichen Fertigungsmaschinen, wie beispielsweise eine Fräsmaschine oder Schleifmaschine im Bereich der Werkshalle, für die Bearbeitung der entnommenen Probe notwendig. Dieses ist als ein besonderer Vorteil anzusehen. Weiterhin ist es gemäß der Erfindung möglich, dass der Zeitaufwand reduziert oder minimiert wird. Es ist weder ein separates Labor, in dem die Probe ausgewertet werden kann, mit irgendwelchen Priorisierungsproblemen noch die Verwendung eines pneumatischen Verschickungssystem oder eines Transportbandes mit auftretenden Restriktionen bei der Verwendung dieser Einreichung notwendig, da die Probe direkt vor Ort, beispielsweise neben den Konverter und der Einheit der verwendeten Lanze im Bereich der Werkshalle mit Hilfe einer Analyseeinheit ausgewertet werden kann. Dieses ist auch als ein weiterer besonderer Vorteil anzusehen. Mittels des Probenhalters ist es möglich, die Probenkammer des Probennehmers einfach, kostengünstig und schnell mit Schmelze zu befüllen. Ferner ist es vorteilhaft möglich, dass der Aufbau des erfindungsgemäßen Probennehmers konstruktiv einfach und kostengünstig ist. Des Weiteren lässt sich der erfindungsgemäße Probennehmer einfach in bestehende Vorrichtungen zur Probenentnahme integrieren.

Das mit der Probenkammer verbundene Einfüllstück, durch das die Schmelze aus dem Schmelzbad in die Probenkammer fließt, ist beispielsweise aus einem Quarzglas, einer Keramik oder dergleichen gebildet.

Der untere, obere und/oder innere Kühlkörper ist vorteilhaft aus einem Metall oder einer Metalllegierung, beispielsweise einer Stahllegierung, ausgestaltet, die bevorzugt einen höheren Schmelzpunkt aufweist als die Schmelze, die die spätere Probe in der Probenkammer bildet.

Es ist ferner alternativ oder ergänzt möglich, dass der Kühlkörper beschichtet ist. Dadurch ist es möglich, beispielsweise eine Oxidation und/oder eine Gefügeveränderung der Probe während des Abkühlens der Probe zu vermeiden, wobei die jeweilige Außenseite der Probe an den zugeordneten, beschichteten Kühlkörper bevorzugt angrenzt.

Eine weitere vorteilhaft Ausgestaltung des Probennehmers ist, dass zumindest der untere Kühlkörper und der innere Kühlkörper eine Wand der Probenkammer bilden, wobei die Wand durch einen Bereich der jeweiligen Außenfläche des unteren Kühlkörpers und des inneren Kühlkörpers gebildet ist, so dass sich zwischen unterem Kühlkörper und innerem Kühlkörper eine Probenkammer mit Hohlraum ausbildet.

Indem sich die abkühlende Probe zwischen unterem Kühlkörper und innerem Kühlkörper befindet, ist es gemäß der Erfindung möglich, die Probe besonders einfach und schnell zu kühlen. Es grenzen nämlich die zugeordnete Fläche des unteren Kühlkörpers und des inneren Kühlkörpers bevorzugt direkt an die Probe an, so dass Wärme direkt über den jeweiligen Kühlkörper abgeleitet werden kann, die dann vom strömenden Gas aus dem Probennehmer abgeführt wird.

Eine weitere vorteilhafte Ausgestaltung des Probennehmers ist, dass der Probennehmer zumindest einen Anschluss für die Zufuhr von Gas, vorzugsweise von einem Inertgas, insbesondere Argon oder Stickstoff, aufweist. Der Anschluss wird auch als Hybridconnector bezeichnet. Mittels des Anschlusses ist es möglich, die von der Probe auf den zugeordneten Kühlkörper, insbesondere den unteren und inneren Kühlkörper übertragene Wärme mit Hilfe eines durch den Anschluss zugeführten Gases abzuführen. Dabei ist es möglich, die Menge an zugeführtem Gas auf den jeweils erwünschten Kühlungseffekt anzupassen. Es wird bevorzugt ein Inertgas, insbesondere Argon oder Stickstoff, verwendet, so dass es zu keiner Reaktion der Probe mit dem zugeführten Gas kommt. Eine weitere bevorzugte Aufgabe des zugeführten Gases durch den Anschluss ist auch, die Probenkammer bis zur Aufnahme der Schmelze in diese frei von der Schmelze zu halten, indem aus dem Einfüllstück des Probennehmers Gas in die Schmelze hinausströmt, so dass keine Schmelze in den Probennehmer zunächst eindringen kann.

Eine weitere vorteilhafte Ausgestaltung des Probennehmers ist, dass zumindest der untere und der innere Kühlkörper voneinander lösbar sind. Durch diese Anordnung ist die Probe einfach aus der Probenkammer, die bevorzugt zwischen unterem Kühlkörper und innerem Kühlkörper gebildet wird, zu entnehmen. Dabei sind die heruntergekühlte Probe und der untere Kühlkörper gemäß einer Ausgestaltung der Erfindung nicht voneinander lösbar, wenn die heruntergekühlte Probe entnommen wird.

Mittels des Spaltes ist es möglich, zum einen eine bestimmte Menge an Gas in den Probennehmer strömen zu lassen. Zum anderen ist es dadurch möglich, eine größere Wärmemenge, die sich beispielsweise nach Aufnahme der Schmelze in die Probenkammer in dem Probennehmer bildet, abzuführen. Die Form des Spaltes kann dabei beliebig räumlich ausgebildet sein, beispielsweise kugelförmig, ellipsenförmig, kegelförmig, trapezförmig und/oder eine Kombination aus diesen. Alternativ oder ergänzend ist auch eine Freiformfläche möglich, die unterschiedliche Ausgestaltungen im Spalt aufweist.

Eine weitere vorteilhafte Ausgestaltung des Probennehmers ist, dass der Probennehmer zumindest eine Gasaustrittsöffnung für die Ausfuhr des zugeführten Gases aufweist. Gemäß vorhergehender Ausführung ist es vor Aufnahme der Schmelze in der Probenkammer möglich, dass zugeführtes Gas aus dem Einfüllstück heraus in die Schmelze strömt. Nachdem durch das Einfüllstück Schmelze in die Probenkammer eingeströmt ist, ist dieser Weg zumindest beeinträchtigt oder versperrt, so dass zugeführtes Gas in gleicher Menge wie vor dem Einfüllen der Schmelze zur Kühlung des Probennehmers anderweitig abgeführt werden muss, um einen Druckanstieg zu vermeiden. Dazu dient eine Gasaustrittsöffnung, die insbesondere nach der Schmelzaufnahme eine Zufuhr von Gas zur Kühlung durch beispielsweise den Spalt zwischen den Kühlkörpern weiterhin ermöglicht und dieses zugeführte Gas dann wieder aus dem Probennehmer herausführt, ohne durch die Probenkammer strömen zu müssen.

In dem Probennehmer weist zumindest einer der Kühlkörper, vorzugsweise der obere Kühlkörper zumindest eine Lüftungsöffnung auf. Die Lüftungsöffnung im Bereich des Kühlkörpers hat den Vorteil, dass die dem Probennehmer zugeführte zur Kühlung verwendete Gasmenge nach der Probennahme durch die Lüftungsöffnung hindurch zur Gasaustrittsöffnung geführt werden kann.

Erfindungsgemäß ist, dass die Lüftungsöffnung mit zumindest einem öffnenbaren Verschluss, vorzugsweise einer öffnenbaren Membran, verschließbar ist, wobei sich der Verschluss während oder nach dem Auffüllen der Schmelze in die Probenkammer öffnet. Gemäß vorhergehender Ausführung ist es vorgesehen, dass zunächst Gas durch die Kühlkörper hindurch, anschließend durch die Probenkammer und anschließend aus dem Einfüllstück herauszuführen, bis es zu einer Aufnahme der Schmelze in die Probenkammer kommt. Nach dem Auffüllen der Schmelze in der Probenkammer ist zumindest teilweise, insbesondere vollständig, der Weg der Strömung des Gases durch das Einfüllstück versperrt, so dass zur Kühlung notwendiges Gas wie vorher nun durch einen öffnenbaren Verschluss hindurch aus dem Probennehmer herausgeführt werden kann. Der Verschluss öffnet sich dabei während oder nach dem Auffüllen der Schmelze in die Probenkammer. Die Öffnung kann dabei mittels Druckanstieg erzeugt werden, indem der Verschluss erst bei einem bestimmten Druck öffnet. Alternativ oder ergänzend ist es möglich, dass der Verschluss durch die Wärmemenge der den Probennehmer umgebenden Schmelze, die nach dem Eintauchen des Probennehmers in die warme, flüssige Schmelze auftritt, für einen Wechsel des Zustandes von geschlossenem Verschluss zu offenem Verschluss beeinflusst wird.

Die Lüftungsöffnung kann beispielsweise eine runde oder eckige Form oder eine Kombination aus beiden haben, beispielsweise eine runde Form mit geraden Anteilen, eine ellipsenförmige Form mit eckigen Anteilen oder dergleichen.

In einer weiteren vorteilhaften Ausgestaltung des Probennehmers weist die Lüftungsöffnung einen Durchmesser von etwa 0,7 mm bis etwa 1,3 mm, vorzugsweise 1,0 mm, auf.

Eine weitere vorteilhafte Ausgestaltung des Probennehmers ist, dass der Verschluss, vorzugsweise die Membran, zumindest eine Kunststoffverbindung, vorzugsweise ein Klebeband, einen Schmelzkleber, einen PVC-Kunststoffstopfen, ein Schließventil mit einer Schmelzverbindung aus Kunststoff oder dergleichen, aufweist. Mittels der Wärme der Schmelze in der Probenkammer ist es möglich, dass der aus einem Kunststoff bestehende Verschluss schmilzt und somit deformiert oder aufgelöst wird, so dass der Verschluss sich teilweise oder vollständig öffnet. Dadurch kann zugeführtes Gas weiterhin in einer vorgesehenen und/oder notwendigen Menge zur Kühlung durch den Probennehmer gemäß vorhergehenden Ausführungen weiter hindurch strömen. Es ist alternativ oder ergänzend möglich, dass durch Einfluss von Druck, der durch die Menge an zugeführtem Gas erzeugt wird, den Verschluss bestehend aus einer Kunststoffverbindung zu öffnen, beispielsweise durch Deformation des Verschlusses.

Das zugeführte Gas wird beispielsweise dann nach Veränderung der Eigenschaften des Verschlusses aus der Lüftungsöffnung oder anschließend aus der Gasaustrittsöffnung herausströmen.

In einer weiteren vorteilhaften Ausgestaltung des Probennehmers weist der Verschluss, vorzugsweise die Membran, eine Druckbeständigkeit von etwa 0,5 bar bis etwa 4 bar, insbesondere zwischen 1,7 bar und etwa 2,3 bar, vorzugsweise etwa 2,0 bar, auf. Es ist somit möglich, dass bei einem bestimmten Druck sich der Verschluss erst öffnet. Ein hoher Druck im Bereich des Verschlusses lässt sich durch eine große Zufuhr von Gas erzeugen, beispielsweise in dem Moment, in dem besonders stark gekühlt werden soll.

Eine weitere vorteilhafte Ausgestaltung des Probennehmers ist, dass der Verschluss, vorzugsweise die Membran, eine Temperaturbeständigkeit von etwa 50 °C bis etwa 90 °C, vorzugsweise etwa 70 °C, aufweist. Dadurch ist es möglich, dass der Verschluss beim Eintauchen des Probennehmers sich nicht schon beim Durchtritt durch die in der Regel auf die Schmelze sich befindenden Schlacke öffnet. Eine Temperaturbeständigkeit führt auch dazu, dass der Verschluss nicht an der Umgebungsluft usw. (und so weiter) öffnet. Beim Einführen des Probennehmers in die Schmelze kommt es zu einem zeitlich verzögerten Anstieg der Temperatur im Bereich des Verschlusses, so dass eine Temperatur von etwa 70 °C an diesem leicht verzögert eintritt, beispielsweise, wenn der Probennehmer schon wieder aus der Schmelze entnommen ist.

In einer besonderen bevorzugten Ausgestaltung ist der Verschluss als Membran ausgestaltet, die eine bestimmte Gasmenge nur hindurch lässt. Die Menge kann dabei beispielsweise abhängig vom Druck und/oder der Umgebungstemperatur um die Membran sein, insbesondere mit Fokus auf die dem Probennehmer zugeführte Wärmemenge und Gasmenge.

Eine weitere vorteilhafte Ausgestaltung ist, dass der Probennehmer zumindest ein Messsystem, vorzugsweise einen Temperatursensor, insbesondere ein Thermoelement, zur Ermittlung der Lage des Probennehmers in der Schmelze aufweist. Dadurch lässt sich die Gaszufuhr in den Probennehmer individuell regeln. Alternativ oder ergänzend ist es dadurch möglich, den Zeitpunkt für den Eintritt der Schmelze durch beispielsweise das Einfüllrohr in die Probenkammer optimal zu bestimmen. Eine Schlackekappe schmilzt bei einer bestimmten Temperatur (z.B. bei 1000°C) und ein Gasstrom in dem Probennehmer kann so geschaltet werden, dass Schmelze in die Probenkammer fließt und eine Probe genommen wird. Alternativ ist es möglich, dass eine Lanze gemäß der Erfindung ein Messsystem, vorzugsweise ein induktives Messsystem, aufweist, wobei der Probennehmer an der Lanze positioniert ist, insbesondere befestigt ist. Zwischen Lanze und Probennehmer ist es möglich, dass ein Probenhalter als Verbindungselement angeordnet ist. Mittels des induktiven Messsystems ist es möglich, die Position des Probennehmers in der Schmelze zu ermitteln. So ist es möglich, den Übergang von Schlacke zu Schmelze zu detektieren und zumindest zu messen, so dass bei oder nach Ermittlung des Übergangs die Gaszufuhr geändert werden kann. So ist es beispielsweise möglich, die Gaszufuhr in den Probennehmer dann zu ändern, wenn der Probennehmer von der Schlacke in die Schmelze eingetaucht worden ist. Das induktive Messsystem weist bevorzugt eine Drahtspule, beispielsweise zur Messung der auftretenden Induktion von Übergang von Schlacke zu Schmelze, auf.

In einer weiteren vorteilhaften Ausgestaltung ist mittels des Spaltes und mittels der Zuführung von Gas die sich aus der Schmelze gebildete Probe in der Probenkammer auf eine Temperatur von etwa 90°C bis etwa 200°C, vorzugsweise etwa 150°C, herunterkühlbar. Gemäß vorhergehenden Ausführungen wird bevorzugt das Gas durch einen Spalt zwischen den jeweiligen Kühlkörpern hindurchgeführt, wodurch eine schnelle und einfache Kühlung von der Schmelztemperatur auf gewünschte Temperaturen, beispielsweise 150°C oder weniger, möglich ist.

Eine weitere vorteilhafte Ausgestaltung ist, dass das Einfüllstück zumindest mittels einer Schutzkappe, vorzugsweise mittels einer Schutzkappe aus Metall, abdeckbar ist. Dieses ermöglicht ein einfaches und schonendes Einführen des Probennehmers in die Schmelze, da diese durch die Schutzkappe abgedeckt ist, die nach Eintritt beispielsweise durch die Schlacke und dann in die Schmelze erst schmilzt. Somit liegt das Einfüllstück für die Aufnahme der Schmelze in die Probenkammer in der Schmelze erst nach dem Schmelzen frei. Es wird dabei jedoch noch Gas vor dem Eintritt der Schmelze in die Probenkammer aus dem Einfüllstück innerhalb der Schmelze herausgeführt.

In einer weiteren vorteilhaften Ausgestaltung ist der Probennehmer an einer Lanze und/oder an einem Trägerstück, vorzugsweise an einem Trägerrohr, insbesondere an einem Probenhalter und einem Trägerrohr, insbesondere einem Trägerrohr aus Pappe, positionierbar. Dadurch ist es möglich, dass der Probennehmer sowohl manuell als auch automatisch in die Schmelze eingeführt werden kann. Insbesondere ist es dadurch möglich, den Probennehmer an einer beliebigen Stelle in der Schmelze zu positionieren. Ferner ist es dadurch möglich, die Lanze wieder zu verwenden, indem ein Trägerrohr verwendet wird, das nach der Aufnahme der Schmelze und nach Abkühlen der Schmelze in der Probenkammer zu einer Probe insoweit beschädigt ist, dass dieses nicht mehr verwendet werden kann. Dabei wird der Probenhalter durch das Trägerrohr selbst geschützt. Es handelt sich somit um einen Wegwerfartikel bei dem Trägerrohr, der einmalig verwendet wird, um insbesondere auch die mehrfach verwendbare Lanze zu schützen. Es ist beispielsweise möglich, auf eine Lanze oder auf einen Probenhalter ein Trägerrohr, das eine bestimmte Länge aufweist, zu positionieren und somit einen bestimmten Abstand zwischen Lanze und Probennehmer zu erzeugen, wenn das Trägerrohr an seinem der Lanze entgegensetzten Ende den Probennehmer aufnimmt. Innerhalb des Trägerrohres befindet sich bevorzugt der Probenhalter, der beispielweise den Probennehmer und die Lanze verbindet. Es werden das Trägerrohr und der Probennehmer so in die Schmelze eingeführt, dass diese in direktem Kontakt mit der Schmelze stehen. Der Probenhalter selbst wird dabei durch das Trägerrohr geschützt. Die Lanze ist gegen die Schmelze auch geschützt.

Es ist es dabei möglich, den jeweiligen Kühlkörper unterschiedlich geometrisch auszugestalten. Es ist beispielsweise möglich, den inneren Kühlkörper in rechteckform, quadratform, scheibenform, dreieckform, pyramidenform, kegelform, kugelform, Kreisform oder dergleichen, beispielsweise einer Kombination aus vorhergehend genannten, auszugestalten. Insbesondere bei den geometrisch zweidimensionalen Formen wie Dreieck, Rechteck, Kreis, Quadrat oder dergleichen weist der innere Kühlkörper auch eine bestimmte Dicke auf, so dass sich eine dreidimensionale räumliche Ausgestaltung des Kühlkörpers ergibt.

Es sind dabei besonders bevorzugt die Formen des unteren Kühlkörpers und des oberen Kühlkörpers an die Form des inneren Kühlkörpers angepasst, so dass eine optimale Kühlung durch sich bildende Spalte im Bereich der Probenkammer und des Probennehmers ergibt. Alternativ oder ergänzend ist es auch möglich, dass die untere oder obere Form des Kühlkörpers die Formgebung des inneren Kühlkörpers beeinflusst.

Durch die Form des Kühlkörpers ist es möglich, die Form des Spaltes zu beeinflussen (oder umgekehrt).

Erfindungsgemäß ist es weiterhin vorgesehen, den Durchmesser oder die Abmessungen des erfindungsgemäßen Spaltes, der besonders bevorzugt zwischen oberem Kühlkörper und innerem Kühlkörper verläuft, auf die jeweiligen notwendigen Mengen an Gas anzupassen. Unter einem Spalt versteht man dabei auch eine flächige Ausgestaltung, beispielsweise ein kegelförmiger Spalt, der einen korrespondierenden kegelförmigen inneren Kühlkörper flächig umschließt. Dadurch ist eine optimale Kühlung der Kühlkörper und somit der Probenkammer und letztendlich des Probennehmers möglich.

Bei dem Verfahren wird zum Beispiel bevorzugt das Gas durch den Anschluss für die Zufuhr von Gas dem Probennehmer zugeführt. Der Anschluss befindet sich bevorzugt innerhalb des Probenhalters. Dadurch ist es einfach und kostengünstig möglich, in den Probennehmer Gas zuzuführen. Insbesondere ist es dadurch möglich, verschiedene Gase, die jeweils auf die Schmelze angepasst sind, an einen solchen Anschluss anzuschließen. Beispielsweise ist es möglich, bei der Schmelze A an den Anschluss des Gas A und bei der Schmelze B an den jeweiligen Anschluss das Gas B oder das Gasgemisch B' anzuschließen.

Bevorzugt strömt das Gas durch zumindest einen Spalt zwischen zumindest inneren Kühlkörper und oberem Kühlkörper. Gemäß vorhergehenden Ausführungen ist dadurch eine optimale und schnelle Kühlung der zu anfangs flüssigen Schmelze in der Probenkammer zur Erzeugung einer verwendbaren Probe möglich. Somit ist eine Entnahme der schnell gekühlten Probe aus der Probenkammer möglich, ohne dass es zu äußeren Einflüssen, wie Oxidationsreaktionen, auf der Probe nach Entfernen dieser aus dem Probennehmer kommt.

Das Gas strömt bevorzugt vor dem Einfüllen der Schmelze nur aus dem Einfüllstück aus und während oder nach dem Auffüllen der Schmelze in die Probenkammer durch zumindest eine Lüftungsöffnung aus. Gemäß vorhergehenden Ausführungen ist es dabei möglich, zu verhindern, dass vor dem eigentlich gewollten Einströmen der Schmelze in die Probenkammer Teile von Schmelze oder Schlacke in die Probenkammer gelangen. Ferner baut sich durch die Lüftungsöffnung kein Druck in dem Probennehmer auf, der die Bildung der flüssigen oder teilweise erstarrten Probe beeinflusst, da entstehender kritischer Druck durch das sich aus der Lüftungsöffnung ausströmende Gas abgeführt wird.

Erfindungsgemäß wird das aus der Lüftungsöffnung ausströmende Gas durch zumindest eine Gasaustrittsöffnung für die Ausfuhr des zugeführten Gases aus dem Probennehmer abgeführt. Das aus der Lüftungsöffnung ausströmende Gas wird durch die Gasaustrittsöffnung abgeführt, die sich beispielsweise in Richtung Lanze befindet. Es ist somit entgegen der Einströmrichtung das Gas wieder aus dem Probennehmer herausführbar.

Dabei wird der Verschluss, vorzugsweise die Membran, während oder nach dem Auffüllen der Schmelze in die Probenkammer mittels der Temperatur der Schmelze und/oder des Druckes des zugeführten Gases gasdurchlässig oder sie wird zerstört. Dadurch ist es möglich, den in den Probennehmer einströmenden Gasstrom so zu regeln, dass eine bestimmte Menge an Gas zugeführt werden kann.

Bevorzugt strömt das Gas nach dem Schmelzen der Schutzkappe, vorzugsweise der Schutzkappe aus Metall, aus dem Einfüllstück aus. Gemäß vorhergehenden Ausführungen ist es dadurch möglich, den Zeitpunkt des Eintritts der Schmelze in die Probenkammer zu beeinflussen.

Nach dem Füllen der Probenkammer mit Schmelze strömt das erneut zugeführte Gas durch den Spalt bevorzugt hindurch und anschließend strömt das Gas aus dem Probennehmer bevorzugt durch die Lüftungsöffnung wieder aus, wobei dadurch die Temperatur der Probe heruntergekühlt wird, vorzugsweise auf eine Temperatur von 90°C bis etwa 200°C, insbesondere etwa 150°C, heruntergekühlt wird. Dadurch ist es auch mit Bezug auf die vorhergehenden Ausführungen möglich, eine schnelle und einfache Kühlung der Probenkammer und somit der darin befindlichen Schmelze oder schon erstarrten Probe zu ermöglichen. Bei einer Temperatur von etwa 150°C ist beispielsweise eine nachfolgende analytische Untersuchung oder mechanische, chemische und/oder elektrische Bearbeitung der Probe möglich, nachdem diese entweder aus der Probenkammer entnommen worden ist oder sich noch in der Probenkammer befindet. Bei 150°C kann beispielsweise die Probe einfach durch der Zerstörung des Probennehmers entnommen werden, ohne die weiteren kritischen Reaktionen aufgrund beispielsweise Umgebungsluft zu erwarten.

Bevorzugt wird die Probe mittels des unteren Kühlkörpers gehalten. Bevorzugt wird alternativ oder ergänzend des Weiteren der innere Kühlkörper mittels des oberen Kühlkörpers gehalten.

Mittels eines Messsystems, vorzugsweise eines Temperaturmesssystems, vorzugsweise eines Temperatursensors, insbesondere eines Thermoelementes, oder eines induktiven Messsystems, wird bevorzugt die Gaszufuhr in den Probennehmer geregelt, insbesondere wird bevorzugt die Gaszufuhr für das Einfüllen der Schmelze in die Probenkammer geändert. Mittels des Messsystems ist es somit möglich, den Zeitpunkt des Einströmens der Schmelze in den Probennehmer und somit in die Probenkammer auf den jeweiligen Anwendungsfall zu regeln, indem mittels des Messsystems ein Zustand erfasst wird, bei dem ein Einströmen der Schmelze erfolgen soll. Ferner lassen sich negative Beeinflussungen, wie das Durchstoßen der Schlacke in Richtung zu messender Schmelze, mittels des Messsystems verringern oder gemäß der Erfindung bevorzugt sogar vermeiden.

Bevorzugt wird die Probe in dem Probennehmer einer Analyseeinrichtung zugeführt. Besonders bevorzugt wird dabei die Probe aus dem Probennehmer, d.h. (das heißt) aus dem zwischen innerem Kühlkörper und unterem Kühlkörper gebildeten Probenkammer, entfernt und anschließend in einer geeigneten Vorrichtung, beispielsweise in einem optischen Emissions-Spektrometer, analysiert. Dabei verbleibt bei der Entnahme der Probe der untere Kühlkörper in einer bevorzugten Ausgestaltung an der Probe.

In einer vorteilhaften Ausgestaltung des Probenhalters gemäß der Erfindung weist der Probenhalter zumindest eine Gasaustrittsöffnung auf, wobei die Ableitung in die Gasaustrittsöffnung endet.

In einer weiteren vorteilhaften Ausgestaltung weist der Probenhalter zumindest einen Zwischenfilter zwischen Schalter und Gasaustrittsöffnung in der Ableitung auf. Der Zwischenfilter ist bevorzugt nach Art eines Gasfilters ausgebildet.

In einer alternativen, vorteilhaften Ausgestaltung des Probenhalters weist die Zuleitung zumindest ein Zuflussventil und/oder die Ableitung zumindest eine Venturidüse auf.

In einer weiteren vorteilhaften Ausgestaltung weist die Ableitung zumindest eine Öffnung, vorzugsweise eine Öffnung im Bereich der Venturidüse, auf.

In einer alternativen, vorteilhaften Ausgestaltung des Probenhalters weist ein in dem Probenhalter angeordneter Teil der mit dem Schalter verbundenen Ableitung ein gegenüber den anderen Teilen der Ableitung vergrößerten Durchmesser auf, der zumindest eine Vakuumkammer bildet, die zumindest eine Gassaugleitung zur Verbindung mit zumindest einer Vakuumpumpe aufweist.

In einer alternativen, vorteilhaften Ausgestaltung des Probenhalters mündet ein in dem Probenhalter angeordneter Teil der mit dem Schalter verbundenen Ableitung in einen hohlen Innenraum des Probenhalters, wobei der Innenraum eine gasdichte Umwandung mit zumindest einer Gassaugleitung zur Verbindung mit zumindest einer Vakuumpumpe aufweist.

In einer weiteren vorteilhaften Ausgestaltung weist die Vakuumkammer ein Volumen zwischen 0,1 l bis etwa 0,5 l, vorzugsweise von etwa 0,3 l, auf.

In einer weiteren vorteilhaften Ausgestaltung weist der Probenhalter und das Kontaktstück jeweils einen Querschnitt mit einem axialsymmetrischen Umfang, insbesondere einem kreisförmigen Umfang, auf.

Bei den Probenhaltern ist zwischen der mit der Probenkammer verbundenen Gasleitung und dem Schalter zumindest ein Gasfilter in einer bevorzugten Ausführungsform angeordnet.

Bei den Probenhaltern weist der Probenhalter zumindest ein Hybridkontaktstück als Kontaktstück und der Probennehmer zumindest einen Hybridconnector in einer bevorzugten Ausgestaltung auf. Das Kontaktstück wird auch als Kontaktblock bezeichnet.

Das Hybridkontaktstück ist vorzugsweise aus einem metallischen Werkstoff und der Hybridconnector ist vorzugsweise aus Kunststoff. Aufgrund der Eigenschaft des Hybridkontaktstück und des bevorzugt korrepondierenden Hybridconnectors, sowohl elektrische Signale als auch pneumatische Signale gleichzeitig oder zeitlich versetzt, durch das jeweilige Hybridbauteil zu leiten, ist eine duale, das heißt hybride Funktion möglich. Das Hybridkontaktstück kann auch zusätzlich eine Hybrideinheit aufweisen, durch die zumindest die Gasleitung und zumindest ein Kabel geführt werden.

Bei der Vorrichtung zur Durchführung von Probenentnahmen weist der Probenhalter in einer bevorzugten Ausführungsform eine in axialer Richtung gemessene Länge von dem Ende des Kontaktstücks zu der gegenüberliegenden Seite des Probenhalters auf und der Schalter ist von dem Ende des Kontaktstücks höchstens 0,3 x Länge, insbesondere 0,1 x Länge, entfernet angeordnet.

Bei der jeweiligen Vorrichtung zur Durchführung von Probenentnahmen sind in einer bevorzugten Ausgestaltung Probennehmer und Probenhalter mit Hilfe eines Trägerstücks, vorzugsweise eines Trägerrohres, insbesondere eines Trägerrohres aus Pappe, verbindbar. Dabei ist der Probennehmer auch selbst mit dem Probenhalter verbindbar.

Bei dem Verfahren zur Probenentnahme aus einer Schmelze strömt in einer Variante bei der Position des Schalters in Stellung B zumindest eine Gasmenge, die sich zumindest in der Probenkammer und dem Einfüllstück befindet, mit Hilfe des Probenhalters gemäß der Erfindung in Richtung Probenhalter, indem die Zufuhr von Gas in die Zuleitung mit Hilfe des Schalters unterbrochen wird.

In einer weiteren, alternativen Verfahrensausgestaltung wird bevorzugt bei der Position des Schalters in der Stellung B zumindest eine Gasmenge, die sich zumindest in der Probenkammer und dem Einfüllstück befindet, mit Hilfe des Probenhalters in Richtung Probenhalter eingesaugt, indem das schon zugeführte Gas in ihrer Richtung mit Hilfe der Venturidüse umgekehrt wird, so dass das zugeführte Gas abgesaugt wird.

In einer weiteren, alternativen Verfahrensausgestaltung wird bei der Position des Schalters in der Stellung B zumindest eine Gasmenge, die sich zumindest in der Probenkammer und dem Einfüllstück befindet, mit Hilfe des Probenhalters in Richtung Probenhalter eingesaugt, in dem das schon zugeführte Gas in ihrer Richtung mit Hilfe des Unterdrucks in der Vakuumkammer umgekehrt wird, so dass das zugeführte Gas abgesaugt wird.

In einer weiteren, alternativen Verfahrensausgestaltung wird bevorzugt bei der Position des Schalters in der Stellung B zumindest eine Gasmenge, die sich zumindest in er Probenkammer und dem Einfüllstück befindet, mit Hilfe des Probenhalters in Richtung Probenhalter eingesaugt, indem das schon zugeführte Gas in ihrer Richtung mit Hilfe des Unterdrucks in der Vakuumkammer umgekehrt wird, so dass das zugeführte Gas abgesaugt wird.

In den Figuren sind bevorzugte Ausführungsformen der Erfindung näher erläutert.

Es zeigen:
- Figur 1: einen Probennehmer in einer besonders bevorzugten Ausführungsform,
- Figur 2: einen Probennehmer in einer alternativen Ausgestaltung,
- Figur 3: einen Probenhalter in einer besonders bevorzugten Ausführungsform,
- Figur 4: einen Probenhalter in einer alternativen Ausgestaltung und
- Figur 5: einen Probenhalter in einer weiteren alternativen Ausgestaltung.

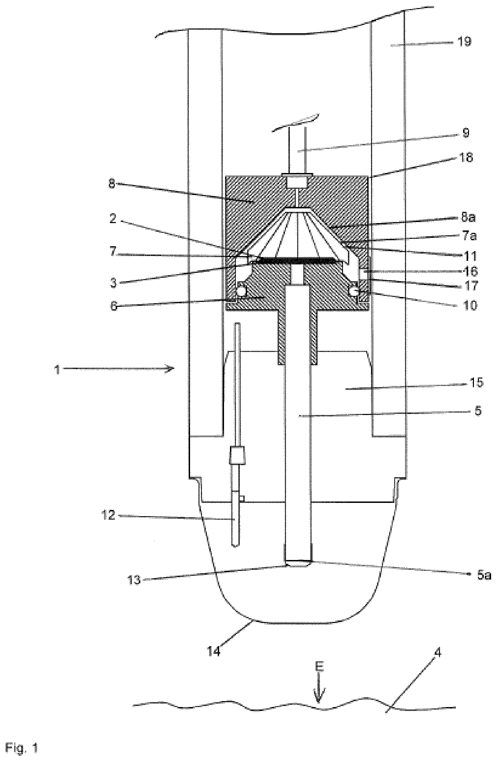

Figur 1 (Fig. 1) zeigt einen Probennehmer 1, der in ein flüssiges und warmes Schmelzbad zur Probennahme getaucht worden war.

Der Probennehmer 1 weist eine Probenkammer 2 auf. In der in Figur 1 dargestellten Probenkammer 2 ist eine Probe 3 beispielhaft dargestellt, die im Ausführungsbeispiel aus einer Schmelze, im Ausführungsbeispiel aus einer Stahlschmelze 4, gebildet ist. Die Stahlschmelze 4 mit einer Temperatur größer als 600 °C ist in Figur 1 beispielhaft als Ausschnitt dargestellt. Ferner weist der Probennehmer 1 ein Einfüllrohr 5 auf, das eine Einfüllöffnung 5a und ein durchgehendes Loch aufweist. Das Einfüllrohr 5 besteht im Ausführungsbeispiel aus Quarzglas. Das Einfüllrohr 5 mündet an dem Probenehmer zugewandten Ende in die Probenkammer und ist mit der Probekammer 2 verbunden.

Gemäß Figur 1 weist der Probennehmer 1 im Ausführungsbeispiel drei Kühlkörper auf, nämlich einen unteren Kühlkörper 6 und einen oberen Kühlkörper 8 und einen inneren Kühlkörper 7. Gemäß dem Ausführungsbeispiel ist die Probenkammer 2 dabei von dem unteren Kühlkörper 6 und dem inneren Kühlkörper 7 unmittelbar umgeben. Der untere Kühlkörper 6 und der innere Kühlkörper 7 umgeben somit die Probenkammer 2 unmittelbar und bilden die innere Wand der Probenkammer 2. Die innere Wand wird somit durch die beiden Kühlkörper 6, 7 gebildet, da deren Außenflächen eine Wand der Probenkammer 2 bilden. Diese Wand wird als innere Wand bezeichnet. Mittels der inneren Wand ist die Probenkammer somit als abgeschlossener Raum zu sehen, in den Schmelze einfließen kann. Die Probekammer 2 ist erfindungsgemäß mittels der Kühlkörper 6, 7, 8 kühlbar.

Der Probennehmer 1 weist gemäß Figur 1 zumindest einen Anschluss 9 für die Zuführung von Gas oder eines Gasgemisches in den Probennehmer 1 auf. Der Anschluss 9 wird auch als Hybridconnector bezeichnet. Im Ausführungsbeispiel wird ein Inertgas, beispielsweise Argon, durch den Anschluss 9 in den Probennehmer 1 zugeführt.

Gemäß Figur 1 hat der innere Kühlkörper 7 die Form nach Art eines Kegels, wobei die Außenflächen des inneren Kühlkörpers 7 Trapezflächen bilden. Der obere Kühlkörper 8 ist an die Form des inneren Kühlkörpers angepasst, so dass dieser gemäß Figur 1 eine korrespondierende Negativform bildet. Der innere Kühlkörper 7 wird dabei im Ausführungsbeispiel mittels des oberen Kühlkörpers 8 gehalten. Der untere Kühlkörper ist an die Form des oberen Kühlkörpers 8 und des inneren Kühlkörpers 7 dahingehend angepasst, dass der obere Kühlkörper 8 eine dichte Verbindung mit dem unteren Kühlkörper 6 an den Berührflachen bildet. An diesen Kontaktflächen von unterem Kühlkörper 6 und oberem Kühlkörpern 8 befindet sich im Ausführungsbeispiel zur Gewährleistung der Dichtheit, insbesondere der Druck- und Gasdichtheit, zusätzlich ein umlaufender O-Ring 10 in einer Nut des unteren Kühlkörpers 6. Gemäß vorhergehenden Ausführungen befindet sich zwischen innerem Kühlkörper 7 und unterem Kühlkörper 6 die Probe 3 in der Probenkammer 2. Die Probe 3 wird mittels des unteren Kühlkörpers 6 festgehalten.

Gemäß Figur 1 sind zumindest der untere Kühlkörper 6 und der innere Kühlkörper 7 voneinander lösbar, so dass die Probe 3 aus dem Probennehmer 1 entnommen werden kann. Dabei verbleibt gemäß dem Ausführungsbeispiel die heruntergekühlte Probe 3 mit dem unteren Kühlkörper 6 fest verbunden, wenn die Probe 3 entnommen wird.

Gemäß Figur 1 weist der Probennehmer 1 zwischen einer Außenwand 7a des inneren Kühlkörpers 7 und der der Außenwand 7a des inneren Kühlkörpers 7 gegenüberliegenden Außenwand 8a des oberen Kühlkörpers 8 zumindest einen Spalt 11 für die Zufuhr von dem im Ausführungsbeispiel verwendeten Inertgas auf. Zwischen den beiden korrespondierenden Kühlkörpern 7, 8 ist somit ein räumlich ausgebildeter Spalt 11 vorhanden.

Der Spalt 11 verläuft dabei zwischen den beiden Außenwänden 7a, 8a, so dass sich ein kegelförmig ausgebildeter Spalt 11 in dem Probennehmer 1 ausbildet. Mittels des in Fig. 1 dargestellten Spaltes 11 und mittels der Zufuhr von Inertgas ist die sich aus der Stahlschmelze 4 gebildete Probe 3 in der Probenkammer 2 im Ausführungsbeispiel auf eine Temperatur von etwa 150 °C zeitlich schnell und einfach herunterkühlbar.

Das Volumen der jeweiligen Kühlkörper 6, 7, 8 ist größer als das Volumen des Spaltes 11 (gemäß Figur 1), vorzugsweise bildet das Volumen des jeweiligen Kühlkörpers 6, 7, 8 in Bezug zum Volumen des Spaltes 11 ein Verhältnis von zumindest 20:1. Dadurch entsteht eine bessere Kühlleistung des Probennehmers 1 gemäß Figur 1.

Der Probennehmer 1 weist im Ausführungsbeispiel des Weiteren ein Messsystem, im Ausführungsbeispiel ein Thermoelement 12 auf, mittels dem die Temperatur und somit die Lage des Probennehmers 1 in der warmen Stahlschmelze 4 ermittelt wird.

In dem in Figur 1 dargestellten Ausführungsbeispiel ist gemäß vorhergehenden Ausführungen der Probennehmer 1 zur Erzeugung einer Probe 3 schon in die Stahlschmelze 4 gehalten worden und nach Erzeugung der Probe 3 in der Probenkammer 2 aus dieser wieder entnommen worden. Die Probe 3 wird dabei in der Probenkammer 2 durch deren innere Wand umschlossen. Es ist folglich im Bereich der Einfüllöffnung 5a des Einfüllrohres 5 die Abdeckung 13 gestrichelt dargestellt, da diese in der Stahlschmelze 4 geschmolzen ist. Ferner ist aus gleichem Grund die im Ausführungsbeispiel verwendete Schutzkappe 14 gestrichelt dargestellt. Sowohl die Abdeckung 13 als auch die Schutzkappe 14 sind nach dem Eintauchen des Probennehmers 1 in die Stahlschmelze 4 geschmolzen. Vor dem Eintauchen in die Stahlschmelze 4 weist der Probennehmer 1 somit eine Abdeckung 13 und eine Schutzklappe 14 auf.

Der Probennehmer 1 weist des Weiteren einen Sandkörper 15 auf, durch den das Einfüllrohr 5 verläuft und in dem sich das Thermoelement 12 befindet. Der Sandkörper 15 hat dabei eine geschlossene Form nach Art eines Sandblockes. Das Einfüllrohr 5 ragt dabei aus dem Sandkörper 15 mit etwas Abstand gemäß Figur 1 heraus. Das Thermoelement 12 steht in direktem Kontakt mit der Stahlschmelze 4. Die Temperaturmessung erfolgt mit Hilfe des Thermoelementes 12 in der Stahlschmelze 4.

Der obere Kühlkörper 8 weist im Ausführungsbeispiel gemäß Figur 1 eine Lüftungsöffnung 16 auf. Die Lüftungsöffnung 16 ist im Ausführungsbeispiel mit einer öffnenbaren Membran 17 verschlossen. Gemäß Figur 1 ist nach dem Einströmen der Stahlschmelze 4 in die

Probenkammer 2 die Membran 17 für Gas geöffnet, wobei sich die Membran im Ausführungsbeispiel zumindest mit dem Auffüllen der Stahlschmelze 4 in die Probenkammer 2 geöffnet hat. Die Membran 17 gemäß dem Ausführungsbeispiel in Figur 1 ist beispielsweise ein Schmelzkleber, der durch die Wärme der Schmelze beeinflusst wird, so dass sich die Membran 17 öffnet. Im Ausführungsbeispiel hat die Lüftungsöffnung 16 einen Durchmesser von 1mm, wobei die Form der Lüftungsöffnung 16 ein rundes Loch ist. Im Ausführungsbeispiel gemäß Figur 1 weist die geschlossene Membran 17 eine Druckbeständigkeit von etwa 2 bar auf und die Temperaturbeständigkeit der Membran 17 ist im Ausführungsbeispiel etwa 70°C.

Des Weiteren weist der Probennehmer 1 im Ausführungsbeispiel eine Gasaustrittsöffnung 18 für die Ausfuhr des zugeführten Gases auf. Bei geöffneter Membran 17 fließt durch die Gasaustrittsöffnung 18 das dem Probennehmer 1 zugeführte Gas wieder aus dem Probennehmer 1 aus.

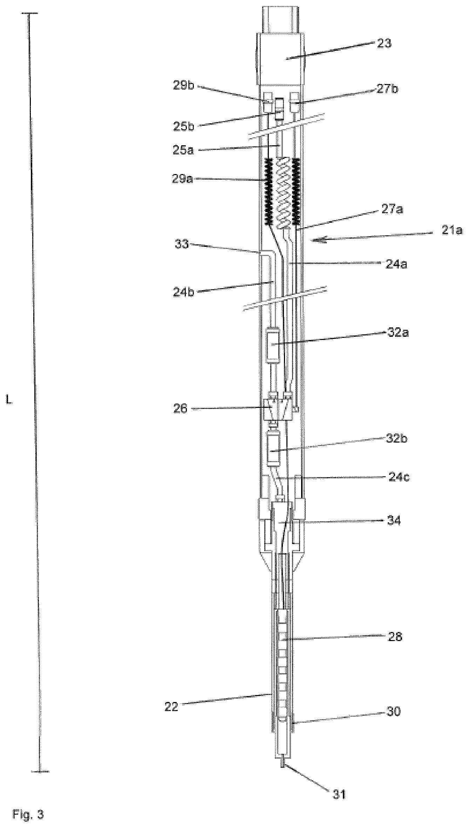

Des Weiteren ist in Figur 1 ein Trägerrohr 19 aus Pappe dargestellt. Der Probennehmer 1 ist an diesem Trägerrohr 19 fest befestigt. Der andere Bereich des Trägerrohr 19 ist an einem in den Figuren 3 bis 5 beispielhaft dargestellten, nachfolgend näher beschriebenen (nicht in Figur 1 dargestellten) Probehalter befestigt und somit zur Probenentnahme aus der Stahlschmelze 4 positioniert. Dieser Probenhalter gemäß einer Ausgestaltung nach den Figuren 3 bis 5 ist somit von dem Trägerrohr 19 aus Pappe umgeben. Der Probennehmer 1 ist somit an einer Seite des jeweiligen Probenhalters mit diesem verbunden.

Die drei Kühlkörper 6, 7, 8 befinden sich gemäß Figur 1 dabei im Bereich des Trägerrohres 19. Im Ausführungsbeispiel nach Figur 1 ist der Probennehmer 1 insbesondere für eine Sublanze (sublance) ausgeführt, so dass der Probennehmer 1 für eine Sublanze und eine zugehörige Vorrichtung verwendet wird. Die Sublanze in Form einer Lanze ist dabei bevorzugt im Bereich der Verbindung Trägerrohr 19 und Probenhalter befestigt.

Nachfolgend wird eine Probenentnahme aus der Stahlschmelze 4 gemäß der Erfindung mit Hilfe des Probennehmers 1 gemäß Figur 1 beispielhaft beschrieben.

Das sich an einem Ende einer nicht dargestellten Lanze befindliche Trägerrohr 19 aus Pappe positioniert den Probennehmer 1 gemäß Figur 1 gemäß vorhergehenden Ausführungen auch mit Hilfe eines gemäß Figuren 3 bis 5 nicht dargestellten Probenhalters. Vor dem Eintauchen des Probennehmers 1 in die Stahlschmelze 4 wird durch den Anschluss 9 das Inertgas in den Probennehmer 1 zugeführt. Das durch den Anschluss 9 zugeführte Gas strömt durch den räumlich ausgebildeten Spalt 11 entlang der Außenwände 7a, 8a zwischen innerem Kühlkörper 7 und oberem Kühlkörper 8 hindurch, nachfolgend durch die leere Probenkammer 2 hindurch in das Einfüllrohr 5, welches vor dem Eintauchen in die Stahlschmelze 4 noch mit einer Abdeckung 13 verschlossen ist. Ferner weist gemäß vorhergehenden Ausführungen der Probennehmer 1 eine Schutzkappe 14 aus Metall auf. Das Gas strömt somit bis in das Einfüllrohr 5 ein. Es bildet sich dabei im Ausführungsbeispiel ein Druck von maximal 2 bar in dem Probennehmer 1 aus, so dass die Membran 17 verschlossen bleibt. Ein Strömungsweg des Gases durch die Lüftungsöffnung 16 hindurch ist folglich aufgrund der noch verschlossenen Membran 17 nicht möglich.

Anschließend wird der Probennehmer 1 in die Stahlschmelze 4 in Eintauchrichtung E eingetaucht. Im Ausführungsbeispiel wird dabei der Probennehmer 1 zunächst durch die Schlacke der Stahlschmelze 4 geführt und anschließend in die Stahlschmelze 4 selbst geführt. Die Position des Probennehmers in der Stahlschmelze 4 ist in Figur 1 nicht dargestellt.

Aufgrund der Wärme der Stahlschmelze 4 schmilzt anschließend die Schutzkappe 14 und ferner die Abdeckung 13. Schutzkappe 14 und Abdeckung 13 sind aus Metall gebildet. Das durch den Anschluss 9 zugeführte Gas strömt folglich aus dem Einfüllrohr 5 in die Stahlschmelze 4 in Richtung Eintauchrichtung E aus dem Probennehmer 1 aus, wodurch jedoch keine Stahlschmelze 4 in das Einfüllrohr 5 eindringen kann. Die drei Kühlkörper 6, 7, 8 zusammen mit der Probenkammer 2 befinden sich oberhalb des Sandkörpers 15, das heißt diese sind in entgegengesetzter Richtung zur Eintauchrichtung E angeordnet. Somit sind diese auch nach dem Eintauchen in die Stahlschmelze 4 geschützt durch das Trägerrohr 19 innerhalb des Schmelzbades.

Mittels des Temperatursensors in Form des Thermoelementes 12 wird die Gaszufuhr in den Probennehmer 1 geregelt, indem gemäß vorhergehenden Ausführungen die Temperatur mittels des Thermoelementes 12 gemessen wird. Gemäß dem Ausführungsbeispiel in Figur 1 wird dabei die Gaszufuhr für das nachfolgende Einfüllen der Stahlschmelze 4 in die Probenkammer 2 bei einer Lage des Probennehmers 1 in der Stahlschmelze 4 in Form einer Änderung unterbrochen, da die Temperatur der Stahlschmelze 4 die Lage des Probennehmers 1 in dieser angibt. Es erwärmt sich dabei unter anderem auch der Sandkörper 15. Bei Erreichen dieser Lage in der Stahlschmelze 4 wird die Gaszufuhr im Ausführungsbeispiel folglich kurzzeitig geändert, so dass nachfolgend sich die Probenkammer 2 mit Stahlschmelze 4 füllen kann. Im Ausführungsbeispiel wird die Gaszufuhr so geändert, dass die Gaszufuhr ausgeschaltet wird. Dabei fließt die Stahlschmelze 4 durch das Loch des Einfüllrohrs in die Probenkammer 2, wobei die Stahlschmelze an der Einfüllöffnung 5a in das Loch eintritt.

Alternativ ist es möglich, statt dem Ausschalten der Gaszufuhr auch einen Unterdruck in der Probenkammer 2 zu erzeugen, so dass die Probenkammer 2 noch schneller sich mit Stahlschmelze 4 füllen kann. Ein Unterdruck ist beispielsweise dadurch erzeugbar, dass an dem Anschluss 9 ein Unterdruck erzeugt wird. Aufgrund der vorhergehend beschriebenen Bauweise des Probennehmers 1 wird dann die Stahlschmelze in die Probenkammer 2 einlaufen und dabei eingesogen durch den Unterdruck.

Nach dem Füllen der Probenkammer 2 mit Stahlschmelze wird der Probennehmer 1 mit Hilfe der Lanze und des Trägerrohres 19 zusammen mit dem Probenhalter aus der Stahlschmelze 4 herausgezogen, so dass der Probennehmer 1 gemäß Figur 1 mit gefüllter Probenkammer 2 vorliegt.

Aufgrund der Temperatur der Stahlschmelze 4 wird die Membran 17 im Ausführungsbeispiel während dem Auffüllen der Stahlschmelze 4 in der Probenkammer 2 gasdurchlässig, da die Temperatur der Stahlschmelze 4 die Membran 17 aufgrund deren Wärmestrahlung beeinflusst oder die Kühlkörper 6;7;8 heizen sich derart auf, dass die Membran 17 zerstört wird. Die vorher geschlossene Membran 17 hat sich somit für Gas geöffnet.

Es ist somit im Ausführungsbeispiel gemäß Figur 1 möglich, dass nach dem Füllen der Probenkammer 2 eine Schmelze 4 und nach dem Herausziehen der Probenkammer 2 aus der Stahlschmelze 4 erneut Gas in den Probennehmer 1 zugeführt wird, so dass die Probe 3 durch das zugeführte Inertgas gekühlt wird. Nachfolgend wird im Ausführungsbeispiel somit die Gaszufuhr in den Probennehmer 1 wieder eingeschaltet.

Indem sich die Probe 3 noch immer in dem Probennehmer 1 befindet und die Probenkammer 2 ausfüllt und somit verschließt, strömt das Inertgas durch den Anschluss 9 hindurch und anschließend durch den kegelförmigen Spalt 11 um den inneren Kühlkörper 7 herum, der an einer Wandseite an die Probe 3 angrenzt. Dabei strömt das Gas gemäß Figur 1 auch aufgrund der geometrischen Ausgestaltung des Spaltes 11 um den unteren Kühlkörper 6 und den oberen Kühlkörper 8 herum, so dass dieser auch (mit-)gekühlt wird. Schließlich strömt das Gas dann aus der Lüftungsöffnung 16 heraus, so dass das aus der Lüftungsöffnung 16 ausströmende Gas durch die Gasaustrittsöffnung 18 aus dem Probennehmer 1 abgeführt wird. Im Bereich der Lüftungsöffnung 16 wird dabei auch die gasdurchlässige Membran 17 durchströmt.

Das erneut zugeführte, die Wärme des Probennehmers 1 aufnehmende und strömende Gas durch den Spalt 11 führt dazu, dass die Temperatur der Probe 3 schnell und einfach heruntergekühlt wird, im Ausführungsbeispiel auf eine Temperatur von etwa 150°C. Ferner führen die Abmessungen des jeweiligen Kühlkörpers 6, 7, 8 und das jeweilige Größenverhältnis von Kühlkörpern 6, 7, 8 zum Spalt 11 zu einer schnellen Wärmeabfuhr.

Bei einer solchen Temperatur von etwa 150°C ist es einfach möglich, dass die Probe 3 aus dem Probennehmer 1 entnommen werden kann und beispielsweise einer Analyseeinrichtung im Ausführungsbeispiel zugeführt wird. Die Analyseeinrichtung ist in Figur 1 nicht dargestellt.

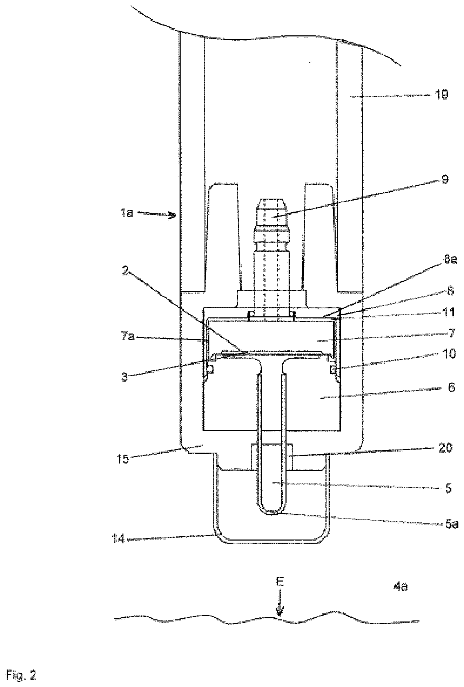

Figur 2 (Fig. 2) zeigt ein weiteres, alternatives Ausführungsbeispiel eines Probennehmers 1a. Dabei werden insbesondere nur die Unterschiede gegenüber dem in Figur 1 dargestellten Probennehmers 1 beschrieben.

Technisch gleiche Bauteile sind mit gleichen Bezugsziffern versehen, neue Bauteile sind mit neuen Bezugsziffern versehen, wobei es möglich ist, dass die geometrische Form der jeweiligen Bauteile zwischen Figur 1 und Figur 2 abweicht
Figur 2 zeigt einen Probennehmer 1a mit einer Probenkammer 2 und einer in der Probenkammer 2 sich aus einer beispielhaft und ausschnittsweise dargestellten Metallschmelze 4a gebildeten Probe 3.

Ferner ist in Figur 2 der untere Kühlkörper 6, der innere Kühlkörper 7 und der obere Kühlkörper 8 dargestellt, die der Probennehmer 1a aufweist. Des Weiteren weist gemäß Figur 2 der Probennehmer 1a einen Anschluss 9 für die Zufuhr von im Ausführungsbeispiel verwendeten Inertgas, insbesondere Argon oder Stickstoff, auf.

Daneben ist der Probennehmer 1a an einem Trägerrohr 19 fest positioniert. Ferner ist an dem Probennehmer 1a ein (nicht in Figur 2 dargestellten) Probenhalter in einer Ausführung gemäß Figuren 3 bis 5 positioniert, wobei das Trägerrohr 19 diesen Probenhalter umgibt. Des Weiteren weist der Probennehmer 1a ein Einfüllohr 5 mit einem Loch auf, welches aus Quarzglas oder Keramik besteht. Das Einfüllrohr 5 weist gemäß Fig. 2 jedoch keine Abdeckung auf.

Die Kühlkörper 6, 7, 8 befinden sich zusammen mit der Probenkammer 2 und der Probe 3 in einem hohlen Sandkörper 15, der eine andere Form aufweist als das Ausführungsbeispiel gemäß Figur 1. Der Sandkörper 15 gemäß Figur 2 umgibt nämlich die Kühlkörper 6, 7, 8 nach Art eines Gehäuses.

Das Einfüllrohr 5 führt dabei aus dem Sandkörper 15 heraus und ist im Bereich der Durchführung teilweise mit Zement 20 fixiert. Das Einfüllohr 5 ragt dabei etwas aus dem hohlen Sandkörper 15 gemäß Figur 2 heraus.

Die drei Kühlkörper 6, 7, 8 befinden sich gemäß vorhergehenden Ausführungen in dem Inneren des Sandkörpers 15. Der untere Kühlkörper 6 ist dabei gegenüber dem inneren Kühlkörper 7 und dem oberen Kühlkörper 8 vom Volumen her größer ausgebildet. Das Volumen des jeweiligen Kühlkörpers 6, 7, 8 in Bezug zum Volumen des Spaltes 11 ist aber zumindest größer als das Volumen des Spaltes 11, vorzugsweise bildet sich ein Verhältnis von zumindest 20:1 aus.

Der innere Kühlkörper 7 weist eine dicke, kreisförmige Scheibenform auf und wird vom oberen Kühlkörper 8 räumlich umschlossen. Durch die geometrische Ausgestaltung ist es möglich, dass der obere Kühlkörper 8 zusätzlich auch in den unteren Kühlkörper 6 eingreift, so dass sich zwischen oberen Kühlkörper 8 und unterem Kühlkörper 6 eine geschlossene Verbindung ergibt, in der der innere Kühlkörper 7 selbst angeordnet ist.

Zum Abdichten von oberem Kühlkörper 8 mit unterem Kühlkörper 6 ist ein O-Ring 10 im Bereich der Kontaktfläche in einer Nut des unteren Kühlkörpers 6 angeordnet. Zwischen dem oberen Kühlkörper 8 und dem inneren Kühlkörper 7 ist gemäß Figur 2 ein räumlicher Spalt 11 in Form einer räumlichen Schale ausgebildet. Es bildet sich eine gas- und druckdichte Anordnung aufgrund der O-Ring-Dichtung und der Geometrie der Kühlkörper 6, 7, 8.

Der innere Kühlkörper 7 weist dabei eine Außenwand 7a auf, die zur Außenwand 8a des oberen Kühlkörpers 8 korrespondiert, so dass sich der Spalt 11 ausbildet, der den gesamten inneren Kühlkörper räumlich umschließt.

Des Weiteren wird die Lage des in Figur 2 dargestellten Probennehmers 1a mit Hilfe eines Messsystems in Form eines nicht dargestellten induktiven Messsystems in der Metallschmelze 4a ermittelt. Mittels des induktiven Messsystems ist es möglich, die Lage des Probennehmers 1a in der Metallschmelze 4a zu messen und somit festzustellen. Dazu befindet sich im Ausführungsbeispiel das induktive Messsystem in der nicht dargestellten Lanze, wobei mittels des Messsystems die Position des Probennehmers 1a in der Metallschmelze 4a ermittelt wird, wenn dieser beispielsweise vollständig in der Metallschmelze 4a eingetaucht ist.

Wie schon zu Figur 1 beschrieben, ist in Figur 2 der Probennehmer 1a nach dem Herausziehen aus der Metallschmelze 4a dargestellt, wobei die Probe 3, die sich aus der Metallschmelze 4a gebildet hat, in der Probenkammer 2 vorliegt. Folglich ist auch die Schutzkappe 14, die der Probennehmer 1a aufweist, gestrichelt dargestellt, da diese in der Metallschmelze 4a geschmolzen war. Vor dem Eintauchen des Probennehmers 1a weist dieser jedoch eine Schutzkappe 14 auf.

Es ist möglich, dass der Probennehmer 1a eine Lüftungsöffnung 16 und eine Gasaustrittsöffnung 18 aufweist. Diese sind in Figur 2 nicht dargestellt.

Der Probennehmer 1a gemäß Figur 2 ist nach Art eines Probennehmers für Roheisenschmelzen (Hot Metal Sampler) ausgeführt.

Zur Herstellung einer Probe 3 in der Probenkammer 2 des Probennehmers 1a gemäß Figur 2 wird die Lanze, auf der das Trägerrohr, der Probenhalter und der Probennehmer 1a positioniert sind, in Eintauchrichtung E in die Metallschmelze 4a eingeführt. Der Probenhalter mit umgebenem Trägerrohr und der Probennehmer 1a befinden sich nach dem Eintauchen vollständig in dem warmen Schmelzbad.

Vor dem Eintauchen wird gemäß vorhergehenden Ausführungen ein Inertgas durch den Anschluss 9 in die Probennehmer 1a zugeführt. Das Gas fließt dabei durch den Spalt 11, anschließend durch die Probenkammer 2, in der sich noch keine Probe 3 befindet, und schließlich durch das Einfüllrohr 5 hindurch in Richtung Schutzkappe 14.

Nach dem Eintauchen des Probennehmers 1a in die Metallschmelze 4a schmilzt die Schutzkappe 14, so dass das zugeführte Gas in die Metallschmelze 4a strömt. Mit Hilfe des induktiven Messsystems wird die Lage des Probennehmers 1a in der Metallschmelze 4a festgestellt, so dass bei unerwünschter Lage die Gaszufuhr gestoppt wird. Es ist dabei alternativ möglich, auch einen Sog mittels Unterdruck in rückwärtiger Richtung gegenüber der vorhergehend beschriebenen Strömungsrichtung des Inertgases aufzubauen, so dass ein Unterdruck in der Probenkammer 2 erzeugt wird, durch den die Metallschmelze 4a besonders einfach und schnell durch das Einfüllrohr 5 hindurch in die Probenkammer 2 einströmt und diese mit Metallschmelze 4a füllt.

Nach dem Füllen der Probenkammer 2 mit Metallschmelze 4a wird der Probennehmer 1a mit Hilfe der Lanze wieder aus der Metallschmelze 4a entgegen der Eintrittsrichtung E herausgeführt.

Im Ausführungsbeispiel wird nach dem Herausführen des Probennehmers 1a aus der Metallschmelze 4a in die Position gemäß Figur 2 erneut Gas durch den Anschluss 9 und den Spalt 11 hindurchgeführt, so dass es zu einer Kühlung des Probennehmers 1a und auch der Probe 3 kommt.

Anschließend ist es möglich, die erstarrte und heruntergekühlte Probe 3 aus dem Probennehmer 1a zu entnehmen, da der untere Kühlkörper 6 und der innere Kühlkörper 7 voneinander lösbar sind. Dabei sind der untere Kühlkörper 6 und die heruntergekühlte Probe 3 gemäß diesem Ausführungsbeispiel nicht voneinander lösbar.

Nachfolgend werden drei Ausgestaltungen eines Probenhalters im Detail beschrieben. Der Probenhalter ist dabei an einen Anschluss 9 des jeweiligen Probennehmers 1, 1a angeschlossen. Gemäß vorhergehenden Ausführungen ist dieser Probenhalter dann von dem Papprohr in Form des Trägerrohres 19 umgeben und auf der gegenüberliegenden Seite zu der Seite des Probennehmers 1, 1a ist der Probenhalter an die jeweils zugehörige Lanze angeschlossen. Das Papprohr umgibt somit den Probenhalter und grenzt jeweils an die Lanze und den Probennehmer 1, 1a an.

Das Ändern der Zufuhr von Gas zum Einfüllen der Schmelze in die Probenkammer 2 lässt sich mit Hilfe der drei beispielhaft ausgeführten Probenhalter gemäß den Figuren 3 bis 5 realisieren. Diese verwenden jeweils unterschiedliche Techniken, um zunächst Gas durch den Anschluss 9 des Probennehmers vor dem Einfüllen hindurchzuführen und anschließende zum Füllen der Probenkammer 2 die Gaszufuhr zu ändern. Dieses wird nachfolgend im Detail beschrieben.

Figur 3 (Fig. 3) zeigt einen Probenhalter 21a zur bevorzugten Aufnahme eines Probennehmers 1, der in Figur 1 dargestellt ist. Es wird bei der Ausgestaltung des Probennehmers 1 gemäß Figur 1 auf vorhergehende Ausführungen verwiesen.

Der Probenhalter 21a weist einen Kontaktblock 22 als Hybridbauteil zur Aufnahme des Probennehmers 1 auf. Der Kontaktblock 22 ist gemäß Figur 3 an einem Ende des Probenhalters 21a angeordnet. Der Kontaktblock 22 korrespondiert zu dem Hybridconnector, das auch als Anschluss 9 des Probennehmers 1 bezeichnet ist, so dass Kontaktblock 22 und Hybridconnector ineinander greifen können. An der gegenüberliegenden Seite des Probenhalters 21a ist eine Aufnahmevorrichtung 23 angeordnet, die im Ausführungsbeispiel ein Gewinde aufweist. Des Weiteren sind in dem Probenhalter 21a mehrere Gasleitungen angeordnet. Der Probenhalter 21a weist im Ausführungsbeispiel gemäß Figur 3 eine Zuleitung 24a, eine Ableitung 24b und eine Gasleitung 24c auf. Die Gasleitung 24c befindet sich dabei auch im Kontaktblock 22. Durch die Zuleitung 24a hindurch ist es möglich, Gas über den Kontaktblock 22 in den nicht in Figur 3 dargestellten Probennehmer 1 einzuleiten. Dabei erfolgt die Einleitung über den Anschluss 9. Die Gasleitung 24c verläuft gemäß Figur 3 durch den Kontaktblock 22 hindurch und ist mit der Probenkammer 2, die in Figur 3 nicht dargestellt ist, somit verbunden, wenn Probennehmer 1 und Probenhalter 21a miteinander verbunden sind. Ferner ist es mit der Ableitung 24b möglich, Gas über den Kontaktblock 22 aus dem nicht dargestellten Probennehmer 1 abzuführen. Im Bereich des einen Ende des Probenhalters 21a, das heißt gemäß Figur 3 im Bereich der Aufnahmevorrichtung 23, ist innerhalb des Probenhalters 21a des Weiteren eine Gasverbindung 25b vorhanden, die mit der Zuleitung 24a verbunden ist. Gemäß Figur 3 ist die Gasverbindung 25b an eine Gaszuführleitung 25a angeschlossen.

Des Weiteren weist der Probenhalter 21a gemäß Figur 3 einen Schalter 26 auf, wobei dieser in dem Probenhalter 21a angeordnet ist und mit der Zuleitung 24a und der Ableitung 24b einerseits und mit der Gasleitung 24c andererseits verbunden ist. Die Änderung des Zustandes des Schalters 26 wird mit Hilfe des Umschaltkabels 27a durchgeführt, wobei am Ende des Umschaltkabels 27a im Bereich der Aufnahmevorrichtung 23 des Probenhalters 21a eine Umschaltkabelverbindung 27b angeordnet ist, an die ein Kabel zum Umschalten ansteckbar ist. Im Bereich des Kontaktblocks 22 weist der Probenhalter 21a des Weiteren Messkontakte 28 auf, die im Bereich des Kontaktblocks 22 angeordnet sind.

Die Messkontakte 28 sind mittels eines Signalkabels 29a verbunden, dessen Ende sich im Bereich der Aufnahmevorrichtung 23 befindet, an dessen Ende eine Signalkabelverbindung 29b angeordnet ist. Im Ausführungsbeispiel sind sechs Messkontakte 28 hintereinander gemäß Figur 3 angeordnet.

Im Bereich des Kontaktblocks 22 ist des Weiteren eine Dichtung 30 angeordnet, so dass beim Verbinden von Probenhalter 21a und nicht dargestelltem Probennehmer 1 eine gasdichte Verbindung zwischen beiden Bauteilen möglich ist. Zwischen Anschluss 9 gemäß Figur 1 und dem Kontaktblock 22 wird somit eine gasdichte Verbindung hergestellt. Der Kontaktblock 22 weist gemäß Figur 3 ferner einen Gasstutzen 31 auf, durch den das Gas durch die Gasleitung 24c hindurch strömen kann.

Gemäß Figur 3 wird die Ableitung 24b durch eine Gasaustrittsöffnung 33 aus dem Probenhalter 21a herausgeführt. Zwischen dieser Gasaustrittsöffnung 33 und dem Schalter 26 ist im Ausführungsbeispiel ein Gasfilter 32a angeordnet. Ein weiterer Gasfilter 32b ist im Bereich der Gasleitung 24c angeordnet.

Der Probenhalter 21a weist des Weiteren eine Hybrideinheit 34 auf, der zwischen Schalter 26 und Gasstutzen 31 angeordnet ist, wobei die Hybrideinheit 34 ermöglicht, dass die Gasleitung 24c und die Signalleitung 29a mit dem nicht dargestellten Probennehmer 1 direkt und fest verbindbar sind. Dazu wird der Kontaktblock 22 gemäß vorhergehenden Ausführungen in den Anschluss 9 passend und gasdicht eingesteckt.

Kennzeichnend für den in Figur 3 dargestellten Probenhalter 21a ist somit, dass der Probenhalter 21a die Gasaustrittsöffnung 33 aufweist, wobei die Ableitung 24b des Probenhalters 21a in die Gasaustrittsöffnung 33 endet. Dabei ist zwischen Schalter 26 und Gasaustrittsöffnung 33 der Ableitung 24b der Gasfilter 32a in Form eines Zwischenfilters angeordnet. Gemäß Figur 3 weist der Probenhalter 21a und der Kontaktblock 22 jeweils einen Querschnitt mit einem kreisförmigen Umfang auf.

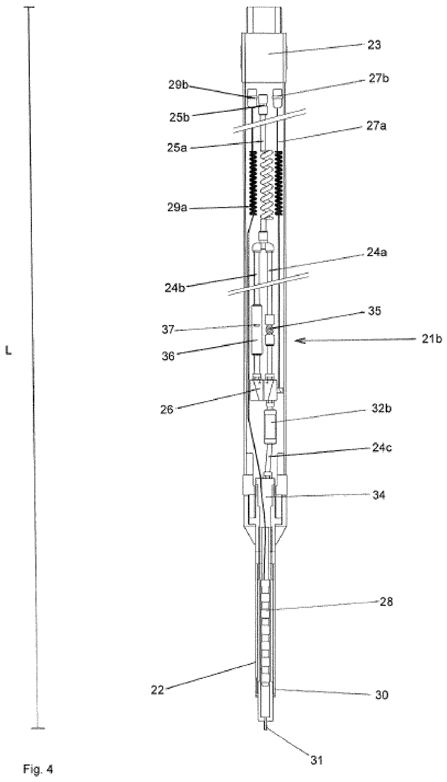

In Figur 4 (Fig. 4) ist eine alternative Ausgestaltung eines Probenhalters 21b dargestellt, wobei nachfolgend gleiche Bauteile mit gleichen Bezugsziffern versehen sind und neue Bauteile mit neuen Bezugsziffern versehen sind.

Nachfolgend werden bei der Figurenbeschreibung zu Figur 4 zunächst die Änderungen gegenüber dem in Figur 3 dargestellten Probenhalter 21a beschrieben. Der in Figur 4 dargestellte Probenhalter 21b weist keine Gasaustrittsöffnung 33 und keinen Gasfilter 32a in Form eines Zwischenfilters auf. Der Probenhalter 21b weist jedoch eine Zuleitung 24a und eine Ableitung 24b auf, die im Bereich des Probenhalters 21b zu einer einzigen Gaszufuhrleitung 25a verbunden sind. Gemäß Figur 4 sind die Zuleitung 24a und die Ableitung 24b mit einem Schalter 26 separat verbunden. In der Zuleitung 24a ist ein Zuflussventil 35 angeordnet und der Ableitung 24b ist gemäß Figur 4 eine Venturidüse 36 angeordnet. Im Ausführungsbeispiel gemäß Figur 4 ist innerhalb der Venturidüse 36 eine Öffnung 37 angeordnet, so dass die Ableitung 24b eine Öffnung 37 im Bereich der Venturidüse 36 aufweist. Die Öffnung 37 ist dabei Teil der Venturidüse 36 und beschreibt eine besondere Ausgestaltung der Venturidüse 36. Die anderen in Figur 4 dargestellten Komponenten des Probenhalters 21b, wie beispielsweise die Aufnahmevorrichtung 23, die Hybrideinheit 34 und der Kontaktblock 22 entsprechen im Aufbau den Komponenten, die schon im Probenhalter 21a gemäß Figur 3 vorhergehend beschrieben wurden. Es ist auf die Ausführungen zu Figur 3 verwiesen und diese ist entsprechend auf die Ausführungen gemäß Figur 4 zu übertragen.

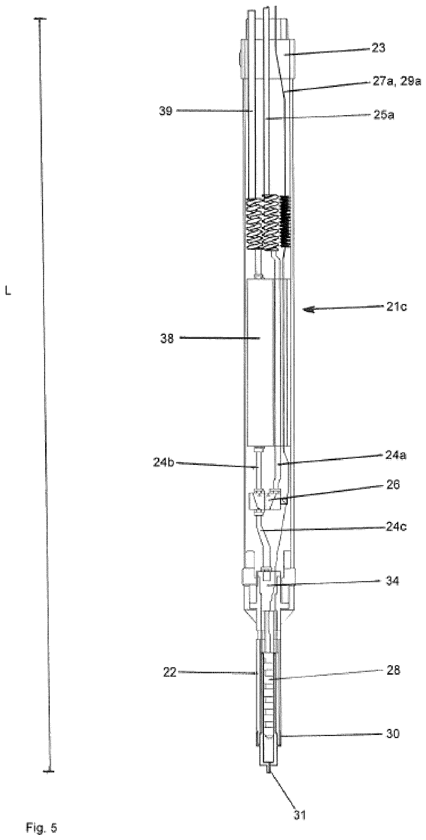

In Figur 5 ist eine weitere alternative Ausgestaltung eines Probenhalters 21c dargestellt, wobei gleiche Bauteile mit gleichen Bezugsziffern versehen sind und neue Bauteile mit neuen Bezugsziffern versehen sind.

Der in Figur 5 (Fig. 5) dargestellte Probenhalter 21c wird nachfolgend so beschrieben, dass die Änderungen gegenüber dem in Figur 3 beschriebenen Probenhalter 21a zunächst nachfolgend beschrieben werden. Der in Figur 5 dargestellte Probenhalter 21c weist keinen Gasfilter 32b und keinen Gasfilter 32a in Form eines Zwischenfilters auf. Ferner weist der Probenhalter 21c keine Gasaustrittsöffnung 33 auf. Des Weiteren weist der in Figur 5 dargestellte Probenhalter 21c keine Verbindungen in Form einer Umschaltkabelverbindung 27b, einer Gasverbindung 25b und einer Signalkabelverbindung 29b auf. Der in Figur 5 dargestellte Probenhalter 21c weist nämlich nur ein aus dem Probenhalter 21c hinausführendes Signalkabel 29a, ein Umschaltbabel 27a und eine Gaszufuhrleitung 25a auf, die jeweils aus dem Probenhalter 21c im Bereich der Aufnahmevorrichtung 23 herausgeführt werden. Diese reichen beispielsweise direkt in die darin anschließende Lanze. Es ist jedoch möglich, dass in diese außerhalb des Probenhalters 21c, beispielsweise innerhalb der Lanze, mit Hilfe einer nicht dargestellten Steckverbindung oder dergleichen an andere Kabel oder Leitungen verbindbar sind.

Innerhalb des Probenhalters 21c ist des Weiteren eine Vakuumkammer 38 angeordnet. Die Vakuumkammer 38 weist dabei im Ausführungsbeispiel ein Volumen von etwa 0,3 l auf. Die Vakuumkammer 38 bildet sich im Probenhalter 21c dadurch, dass ein in dem Probenhalter 21c angeordneter Teil der mit dem Schalter 26 verbundenen Ableitung 24b ein gegenüber den anderen Teilen der Ableitung 24b vergrößerten Durchmesser aufweist. In dem vergrößerten Durchmesser selbst bildet sich dabei somit die Vakuumkammer 38. Die Vakuumkammer 38 ist dabei mit einer Gassaugleitung 39 als weitere, zusätzlich vorhandene Leitung verbunden, wobei die Gassaugleitung 39 mit einer nicht dargestellten Vakuumpumpe verbunden ist.

Die anderen in Figur 3 beschriebenen Komponenten des Probenhalters 21a sind auch bei dem Probenhalter 21c vorhanden und werden nicht gemäß vorgehenden Ausführungen erneut beschrieben. Diese sind entsprechend übertragbar.

Die jeweils in den Figuren 3 bis 5 beschriebenen Probenhalter 21a, 21b, 21c sind beispielsweise in einer Vorrichtung zur Durchführung von Probennahmen in Metallschmelzen mit einer Lanze, insbesondere in Stahlschmelzen mit einer Sublanze, verwendbar. In den Figuren 3 bis 5 ist eine solche Vorrichtung sowie eine zugehörige Lanze, insbesondere eine Sublanze, nicht dargestellt. Es ist aber hinreichend bekannt, dass eine Lanze einen Lanzenkörper aufweist, die in einer solchen Vorrichtung angeordnet ist.

Es ist dabei vorgesehen, dass an einem Ende des Lanzenkörpers ein Probenhalter 21a, 21b, 21c gemäß einem der Ausführungen zu Figur 3 bis 5 verbindbar ist, mit dem insbesondere ein Probennehmer 1 gemäß Figur 1 für die Verwendung in einer Stahlschmelze verbindbar ist. Die Vorrichtung weist im Ausführungsbeispiel mit Hilfe des jeweiligen Probenhalters 21a, 21b, 21c gemäß den Figuren 3 bis 5 eine Zuleitung 24a zur Einleitung von Gas über den Kontaktblock 22 in den Probennehmer 1 und einer Ableitung 24b zum Absaugen von Gas über den Kontaktblock 22 aus dem Probennehmer 1 und eine mit der Probenkammer 2 verbundene Gasleitung 24c auf.

Der in einer solchen nicht in den Figuren 1 bis 5 dargestellten Vorrichtung verwendete Probenhalter 21a, 21b, 21c weist eine in axialer Richtung gemessene Länge L von dem Kontaktblock 22 zu der gegenüberliegenden Seite des Probenhalters 21a, 21b, 21c auf. Der Schalter 26 in dem jeweiligen Probenhalter 21a, 21b, 21c ist dabei von dem Ende des Kontaktblocks 22 im Ausführungsbeispiel 0,1 x seiner Länge L entfernt angeordnet.

Gemäß der vorhergehenden Ausführungen zu den Figuren 3 bis 5 ist der Probennehmer 1 und der Probenhalter 21a, 21b, 21c mit Hilfe eines Trägerrohres 19 aus Pappe verbindbar, wobei das Trägerrohr 19, das im Bereich der Dichtung 30 am Kontaktblock 22 an den Probennehmer 1 selbst angesetzt wird, gemäß vorgehenden Ausführungen nicht in den Figuren 3 bis 5 dargestellt ist.

Zur Entnahme einer Probe 3 aus einem Probennehmer 1 gemäß Figur 1, die aus einer Stahlschmelze 4 gebildet worden ist, wird nachfolgend das zugehörige Verfahren mit einem Probenhalter 21a gemäß Figur 3 und einem Probennehmer 1 gemäß Figur 1 als besonders bevorzugte Ausführungsform beschrieben.

Dazu wird eine nicht in Figur 3 dargestellte Sublanze mit dem Probenhalter 21a verbunden, wobei die Verbindung im Bereich der Aufnahmevorrichtung 23 erfolgt. Die zugehörigen Signalkabelverbindung 29b, die Umschaltkabelverbindung 27b und die Gasverbindung 25b sind jeweils mit korrespondierenden Anschlüssen innerhalb der Sublanze verbunden. Im Bereich des Kontaktblocks 22 des Probenhalters 21a ist der Probennehmer 1 gemäß Figur 1 positioniert, wobei der Gasstutzen 31 so in dem Anschluss 9 des Probennehmer 1 angeordnet ist, dass eine gasdichte Verbindung zwischen Probenhalter 21a und Probennehmer 1 entsteht. Zwischen Probennehmer 1 und Aufnahmevorrichtung 23 des Probenhalters 21a als Teil des Probenhalters 21a wird ein Trägerrohr 19 aus Pappe positioniert, so dass zwischen Probennehmer 1 und Ende des Trägerrohres 19 sich der Probenhalter 21a befindet. Das Trägerrohr 19 ist mit der Aufnahmevorrichtung 23 fest verbunden, beispielsweise mit Hilfe einer Eingreifverbindung nach Art eines Gewindes, wobei sich dann beispielsweise die Aufnahmevorrichtung 23 durch eine wellige Oberfläche in das Trägerrohr 19 fest eindrückt.

Der Probennehmer 1 wird anschließend in dieser Vorrichtung in eine in Figur 1 angedeutete Stahlschmelze eingetaucht, wobei vor dem Eintauchen durch die Zuleitung 24a, die durch die Gaszufuhrleitung 25a mit einem Inertgas durchströmt wird, das durch die Sublanze vorab zugeführt worden ist. Das durch die Zuleitung 24a zugeführte Inertgas wird anschließend durch den Schalter 26 geführt, wobei sich dieser in der Stellung A befindet, und somit in die Gasleitung 24c geführt, wobei auch im Bereich der Gasleitung 24c sich gemäß vorhergehenden Ausführungen die Hybrideinheit 34 befindet. Beim Durchströmen des Inertgases durch den Gasstutzen 31 in den Probennehmer 1 gemäß den Ausführungen zu Figur 1 tritt schließlich das Gas zunächst nur in das Einfüllrohr 5 ein. Nach dem Eintauchen zumindest des Probennehmers 1 in die Stahlschmelze 4 und dem Aufschmelzen der Schutzkappe 14 sowie der Abdeckung 13 gemäß den Ausführungen zu Figur 1 tritt Gas aus dem Einfüllrohr 5 aus, wenn der Probennehmer 1 sich in der Stahlschmelze 4 befindet. Gleichzeitig wird über das Signalkabel 29, das auch durch die Hybrideinheit 34 in das Thermoelement 12 des Probennehmers 1 geführt ist, die Temperatur der Stahlschmelze und auch die Position des Probennehmers 1 in der Stahlschmelze gemessen und ausgewertet, wobei die Auswertung über eine nicht dargestellte externe Einheit verläuft, die die mit Hilfe des Signalkabels 29 durch Probenhalter 21a und nicht dargestellte Sublanze übertragenen Daten auswertet.

Nach Erreichen der geeigneten Position des Probennehmers 1 in der Stahlschmelze 4 wird die Gaszufuhr durch die Zuleitung 24a gemäß den vorhergehenden Ausführungen zu Figur 1 unterbrochen, indem der Schalter 26 in die Stellung B umgeschaltet wird. Dadurch wird die Zufuhr von Gas durch die Zuleitung 24a unterbrochen. Im Ausführungsbeispiel wird der Schalter 26 in dem Probenhalter 21a so umgeschaltet, dass sich nachfolgend die Probenkammer 2 des Probennehmers mit Stahlschmelze 4 füllt. Anschließend wird der Probennehmer 1 sowie das Trägerrohr 19 und der Probenhalter 21a aus der Stahlschmelze mit Hilfe der beweglichen Sublanze in der Vorrichtung wieder entnommen, nachdem sich die Probenkammer 2 mit Schmelze vollständig gefüllt hat. Zum Kühlen des Probennehmers 1 und der Probenkammer 2 wird anschließend durch Umschalten des Schalters 26 von der Stellung B in die Stellung C, die im Ausführungsbeispiel gemäß Figur 3 der Stellung A entspricht, erneut umgeschaltet bzw. zurückgeschaltet. Dadurch ist es möglich, dass die Probenkammer 2 durch das zugeführte Gas gekühlt wird. Die Kühlung ist im Detail im Ausführungsbeispiel zu Figur 1 beschrieben. Die Umschaltung des Schalters 26 erfolgt mittels des Umschaltkabels 27a, durch das der Schalter 26 jeweils umschalten lässt.

Beim Umschalten des Schalters 26 von der Stellung A in die Stellung B tritt Gas durch den Probennehmer 1 in den Probenhalter 21a aus, wobei das Gas dann durch die Gasleitung 24c und die Hybrideinheit 34 strömt, anschließend den Gasfilter 32b durchströmt und schließlich den Schalter 26 durchgeführt. Durch die Schalterstellung B tritt das Gas dann durch den weiteren Gasfilter 32a in Form eines Zwischenfilters durch die Ableitung 24b durch die Gasaustrittsöffnung 39 aus. Aufgrund dieses Gasaustritts ist es möglich, dass sich die Probenkammer 2 mit Schmelze füllt. Ausführlich ist dieses im Ausführungsbeispiel zu Figur 1 beschrieben. Es wird dabei kein Vakuum oder Unterdruck gemäß vorhergehenden Ausführungen erzeugt. Es tritt somit zumindest die Gasmenge, die sich in der Probenkammer 2 und dem Einfüllstück 5 befindet, aus der Gasaustrittsöffnung 39 aus.

Durch die Messung einer Temperatur mit Hilfe des Thermoelementes 12 von beispielsweise 1100 °C wird der Schalter 26 von der Stellung A in die Stellung B geschaltet. Alternativ oder ergänzend ist es möglich, die Lanzenposition elektrisch oder durch Druck in der Stahlschmelze mit Hilfe der Position des Probennehmers 1 zu messen.

Die vorhergehend beschriebene Probenentnahme aus einer Stahlschmelze 4 lässt sich auch mittels eines Probenhalters 21b gemäß Figur 4 realisieren.

Gemäß der Ausführung zu Figur 3 gemäß vorhergehenden Ausführungen wird der Probenhalter 21a in einer weiteren Ausgestaltung dabei durch den Probenhalter 21b ersetzt. Nachfolgend wird das Durchströmen des Gases durch den Probenhalter 21b beschrieben, und Unterschiede gegenüber der vorhergehenden Ausführung mit dem Probenhalter 21a herausgestellt.

Nachdem die jeweiligen Anschlüsse gemäß Figur 4 passend an die Sublanze oder den Probennehmer gemäß Figur 1 angeschlossen sind, strömt das Inertgas durch die Gaszufuhrleitung 24d in die Zuleitung 24a und die Ableitung 24b. Das durch Gaszuführleitung 25a fließende Gas strömt dann zum einen durch das Zuflussventil 35 in den Schalter 26, der sich in der Stellung A befindet. Das zugeströmte Gas kann somit durch den Schalter 26 und den Gasfilter 32b durch die Hybrideinheit 34 hindurch in den Probennehmer 1 einströmen. Gleichzeitig strömt zu anderen durch die Ableitung 24b Gas, das aus der Gaszufuhrleitung 25a zugeführt wird, durch die Venturidüse 36 hindurch, so dass zwischen Venturidüse 36 und Schalter 26 ein Unterdruck aufgrund der besonderen Ausführung der Venturidüse 36 entsteht. Das Gas, das durch die Ableitung 24b zugeführt wird, wird dabei durch die Öffnung 37 ausgeführt.

Zum Füllen der Probenkammer 2 wird der Schalter 26 von der Stellung A in die Stellung B umgeschaltet, so dass das durch die Zuleitung 24a strömende Gas aufgrund der Schalterstellung B nicht mehr in die Gasleitung 24c strömen kann. Ausschließlich das durch die Gaszufuhrleitung 25a strömende Gas kann durch die Ableitung 24b und die Venturidüse 36 durch die Öffnung 37 entweichen, wobei dabei ein Unterdruck weiterhin zwischen Venturidüse 36 und Schalter 26 aufgebaut wird, der in die Gasleitung 24c übertragen wird. Folglich entsteht ein Unterdruck in der Probenkammer 2, die zum Einsaugen von Schmelze mit Hilfe der Venturidüse 36 in die Probenkammer 2 entsteht. Nach Füllen der Probenkammer 2 mit Schmelze wird der Schalter 26 zurück in die Stellung A geschaltet, so dass die Probenkammer 2 mit Hilfe des durch die Zuleitung 24a zugeführten Gases gekühlt werden kann.

Die Entnahme der Probenkammer bzw. des Probennehmers 1 aus der Schmelze wurde vorhergehend im Detail beschrieben.

Die vorhergehend beschriebene Probenentnahme mit Hilfe eines Probenhalters 21a aus einer Stahlschmelze 4 lässt sich auch mittels eines Probennehmers 21c gemäß Figur 5 realisieren. In einer solchen Vorrichtung wird nachfolgend ausschließlich der zwischen Probennehmer 1 und Sublanze besonders ausgeführte Probenhalter 21c im Detail beschrieben und Änderungen oder andere technische Umsetzungen im Detail herausgestellt.

Durch die Zuleitung hindurchströmt gemäß Figur 5 ein Inertgas aus der Sublanze durch den Schalter 26 hindurch und in die Gasleitung 24c in den Probennehmer 1 gemäß Figur 1. Zum Füllen der Probenkammer 2 wird der Schalter 26 von der Stellung A in die Stellung B geschaltet, so dass das vorhergehend ausschließlich durch die Zuleitung 24 zugeführte Inertgas blockiert wird, da der Schalter 26 in die Stellung B geschaltet worden ist. Folglich kann Gas durch die Gasleitung 24c durch den Schalter 26 hindurch in die Vakuumkammer 38 einströmen, in der ein Unterdruck vorherrscht. Dieser wurde beispielsweise über die Gassaugleitung 39 mit Hilfe einer Vakuum-Pumpe vorab in der Vakuumkammer 38 erzeugt. Folglich wird die Schmelze 4 in die Probenkammer 2 mit Hilfe des Unterdrucks aus der Vakuumkammer 38 eingesogen, wenn der Schalter 26 von der Stellung A in die Stellung B umgeschaltet worden ist. Zum Kühlen wird der Schalter 26 zurück in die Stellung A geschaltet, so dass wieder Inertgas durch die Zuleitung 24a und anschließend durch die Gasleitung 24c in den Probennehmer 1 einströmen kann, so dass die Probenkammer 2 gekühlt wird.

Das Eintauchen der jeweiligen Vorrichtungen, insbesondere bezüglich der Probenhalter 21b und 21c, wurde im Detail für den Probenhalter 21a beschrieben und ist entsprechend auf die Probenhalter nach 21b, 21c übertragbar. Ferner wurde das Herausziehen des Probennehmers 1 gemäß Figur 1 aus der Schmelze 4 im Detail beschrieben, so dass dieses auch auf die Probenhalter 21b und 21c übertragbar ist.

## Patentansprüche

1. Probennehmer (1, 1a) mit einer Probenkammer (2) für eine sich aus einer Schmelze bildende Probe (3) aufweisend zumindest einen unteren Kühlkörper (6), zumindest einen oberen Kühlkörper (8) und zumindest einen inneren Kühlkörper (7) und zumindest ein Einfüllstück, wobei die Probenkammer (2) zumindest von dem unteren Kühlkörper (6) und von dem inneren Kühlkörper (7) gemeinsam umgeben ist, so dass zumindest die Probenkammer (2) zumindest mittels des unteren und inneren Kühlkörpers (6, 7) kühlbar ist, wobei das mit der Probenkammer (2) verbundene Einfüllstück mit einer Einfüllöffnung (5a) in die Probenkammer (2) mündet und wobei die Kühlkörper (6, 7, 8) jeweils eine Außenfläche (7a, 8a) aufweisen, **dadurch gekennzeichnet, dass** der Probennehmer (1, 1a) zwischen einem Bereich der Außenfläche (7a) des inneren Kühlkörpers (7) und dem diesem Bereich der Außenfläche (7a) des inneren Kühlkörpers (7) gegenüberliegenden Bereich der Außenfläche (8a) des oberen Kühlkörpers (8) zumindest einen Spalt (11) für die Durchleitung von zumindest einem Gas aufweist und dass das Volumen des jeweiligen Kühlkörpers (6, 7, 8) größer als das Volumen des Spaltes (11) ist, dass zumindest einer der Kühlkörper (6, 7, 8) zumindest eine Lüftungsöffnung (16) aufweist und dass die Lüftungsöffnung (16) mit zumindest einem öffnenbaren Verschluss, vorzugsweise einer öffnenbaren Membran (17), verschließbar ist, wobei sich der Verschluss während oder nach dem Auffüllen der Schmelze in die Probenkammer (2) öffnet.

2. Probennehmer (1, 1a) nach Anspruch 1, **dadurch gekennzeichnet, dass** zumindest der untere Kühlkörper (6) und der innere Kühlkörper (7) eine Wand der Probenkammer (2) bilden, wobei die Wand durch einen Bereich der jeweiligen Außenfläche des unteren Kühlkörpers (6) und des inneren Kühlkörpers (7) gebildet ist, so dass sich zwischen unterem Kühlkörper (6) und inneren Kühlkörper (7) eine Probenkammer (2) mit Hohlraum ausbildet.

3. Probennehmer (1, 1a) nach Anspruch 1 oder Anspruch 2, **dadurch gekennzeichnet, dass** die Lüftungsöffnung (16) einen Durchmesser von etwa 0,7 mm bis etwa 1,3 mm aufweist.

4. Probennehmer (1, 1a) nach einem der Ansprüche 1 oder 3, **dadurch gekennzeichnet, dass** der Verschluss, vorzugsweise die Membran (17), zumindest eine Kunststoffverbindung aufweist.

5. Probennehmer (1, 1a) nach einem der Ansprüche 1 bis 4, **dadurch gekennzeichnet, dass** der Verschluss eine Druckbeständigkeit von etwa 0,5 bar bis etwa 4 bar aufweist.

6. Probennehmer (1, 1a) nach einem der Ansprüche 1 bis 5, **dadurch gekennzeichnet, dass** der Verschluss eine Temperaturbeständigkeit von etwa 50°C bis etwa 90°C aufweist.

7. Verfahren zur Probenentnahme aus einer Schmelze mit einer Schmelztemperatur von größer als 600 °C, wobei der an einem Ende einer Lanze und/oder eines Trägerstücks positionierte Probennehmer (1, 1a) gemäß einem der Ansprüche 1 bis 6 in die Schmelze eingetaucht wird, wobei anschließend die Probenkammer (2) des Probennehmers (1, 1a) mit Schmelze gefüllt wird und wobei danach zumindest die Probe (3) mit Hilfe des Probennehmers (1, 1a) aus der Schmelze herausgezogen wird, **dadurch gekennzeichnet, dass** vor dem Eintauchen zumindest ein Gas in den Probennehmer (1, 1a) zugeführt wird, wobei das Gas durch zumindest ein Einfüllstück aus dem Probennehmer (1, 1a) wieder ausströmt, anschließend der Probennehmer (1, 1a) in die Schmelze eingetaucht wird, danach die Zufuhr von Gas geändert wird, nachfolgend sich die Probenkammer (2) mit Schmelze füllt, nachfolgend während oder nach dem Füllen der Probenkammer (2) mit Schmelze erneut Gas in den Probennehmer (1, 1a) zugeführt wird, so dass zumindest die Probenkammer (2) durch das zugeführte Gas gekühlt wird, dass das aus der Lüftungsöffnung (16) ausströmende Gas durch zumindest eine Gasaustrittsöffnung (18) für die Ausfuhr des zugeführten Gases aus dem Probennehmer (1, 1a) abgeführt wird und dass der Verschluss während oder nach dem Auffüllen der Schmelze in die Probenkammer (2) mittels der Temperatur der Schmelze und/oder des Druckes des zugeführten Gases gasdurchlässig wird.

8. Verfahren nach Anspruch 7, **dadurch gekennzeichnet, dass** das Gas durch zumindest einen Spalt (11) zwischen zumindest innerem Kühlkörper (7) und oberen Kühlkörper (8) strömt.

9. Verfahren nach Anspruch 7 oder 8, **dadurch gekennzeichnet, dass** das Gas vor dem Einfüllen der Schmelze nur aus dem Einfüllstück ausströmt und während oder nach dem Auffüllen der Schmelze in die Probenkammer (2) durch zumindest eine Lüftungsöffnung (16) ausströmt.

10. Verfahren nach einem der vorhergehenden Ansprüche, **dadurch gekennzeichnet, dass** nach dem Füllen der Probenkammer (2) mit Schmelze das erneut zugeführte Gas durch den Spalt (11) hindurchströmt und anschließend das Gas aus dem Probennehmer (1, 1a) durch die Lüftungsöffnung (16) wieder ausströmt, wobei dadurch die Temperatur der Probe (3) heruntergekühlt wird.

11. Verfahren nach einem der vorhergehenden Ansprüche, **dadurch gekennzeichnet, dass** die Probe (3) mittels des unteren Kühlkörpers (6) gehalten wird und/oder dass der innere Kühlkörper (7) mittels des oberen Kühlkörpers (8) gehalten wird.

12. Verfahren nach einem der vorhergehenden Ansprüche, **dadurch gekennzeichnet, dass** die Probe (3) in dem Probennehmer (1, 1a) einer Analyseeinrichtung zugeführt wird.

## Claims

1. A sampler (1, 1a) comprising a sample chamber (2) for a sample (3) forming from a molten material, having at least one lower cooling body (6), at least one upper cooling body (8), and at least one inner cooling body (7), and at least one filling part, wherein the sample chamber (2) is surrounded jointly at least by the lower cooling body (6) and by the inner cooling body (7), so that at least the sample chamber (2) can be cooled by at least the lower and inner cooling body (6, 7), wherein the filling part, which is connected to the sample chamber (2), leads into the sample chamber (2) with a filling opening (5a), and wherein the cooling bodies (6, 7, 8) each have an outer surface (7a, 8a), **characterised in that**, between a region of the outer surface (7a) of the inner cooling body (7) and the region of the outer surface (8a) of the upper cooling body (8) located opposite said region of the outer surface (7a) of the inner cooling body (7), the sampler (1, 1a) has at least one gap (11) for the guide-through of at least one gas, and that the volume of the respective cooling body (6, 7, 8) is larger than the volume of the gap (11), that at least one of the cooling bodies (6, 7, 8) has at least one ventilation opening (16), and that the ventilation opening (16) can be closed by means of at least one closure, which can be opened, preferably a membrane (17), which can be opened, wherein the closure opens while or after the molten material is filled into the sample chamber (2).

2. The sampler (1, 1a) according to claim 1, **characterised in that** at least the lower cooling body (6) and the inner cooling body (7) form a wall of the sample chamber (2), wherein the wall is formed by a region of the respective outer surface of the lower cooling body (6) and of the inner cooling body (7), so that a sample chamber (2) comprising a hollow space forms between lower cooling body (6) and inner cooling body (7)

3. The sampler (1, 1a) according to claim 1 or claim 2, **characterised in that** the ventilation opening (16) has a diameter of approximately 0.7 mm to approximately 1.3 mm.

4. The sampler (1, 1a) according to any one of claims 1 or 3, **characterised in that** the closure, preferably the membrane (17), has at least one plastic connection.

5. The sampler (1, 1a) according to any one of claims 1 to 4, **characterised in that** the closure has a pressure resistance of approximately 0.5 to approximately 4 bar.

6. The sampler (1, 1a) according to any one of claims 1 to 5, **characterised in that** the closure has a temperature resistance of approximately 50°C to approximately 90°C.

7. A method for sampling from a molten material with a melting temperature of more than 600°C, wherein the sampler (1, 1a) according to one of claims 1 to 6, which is positioned at an end of a lance and/or of a carrier part, is immersed into the molten material, wherein the sample chamber (2) of the sampler (1, 1a) is subsequently filled with molten material, and wherein at least the sample (3) is then pulled out of the molten material with the help of the sampler (1, 1a), **characterised in that** at least one gas is supplied into the sampler (1, 1a) prior to the immersion, wherein the gas flows out of the sampler (1, 1a) again through at least one filling part, the sampler (1, 1a) is subsequently immersed into the molten material, the supply of gas is then changed, the sample chamber (2) subsequently fills with molten material, gas is subsequently supplied into the sampler (1, 1a) again during or after filling the sample chamber (2) with molten material, so that at least the sample chamber (2) is cooled by means of the supplied gas, that the gas flowing out of the ventilation opening (16) is discharged through at least one gas outlet opening (18) for discharging the supplied gas out of the sampler (1, 1a), and that the closure becomes gas-permeable during or after the molten material is filled into the sample chamber (2) by means of the temperature of the molten material and/or of the pressure of the supplied gas.

8. The method according to claim 7, **characterised in that** the gas flows through at least one gap (11) between at least inner cooling body (7) and upper cooling body (8).

9. The method according to claim 7 or 8, **characterised in that** the gas only flows out of the filling part before the molten material is filled in, and flows out through at least one ventilation opening (16) during or after the molten material is filled into the sample chamber (2).

10. The method according to any one of the preceding claims, **characterised in that**, after filling the sample chamber (2) with molten material, the newly supplied gas flows through the gap (11), and the gas subsequently flows out of the sampler (1, 1a) again through the ventilation opening (16), wherein the temperature of the sample (3) is thus cooled down.

11. The method according to any one of the preceding claims, **characterised in that** the sample (3) is held by means of the lower cooling body (6) and/or that the inner cooling body (7) is held by means of the upper cooling body (8).

12. The method according to any one of the preceding claims, **characterised in that** the sample (3) in the sampler (1, 1a) is supplied to an analysis device.

## Revendications

1. Dispositif de prélèvement d'échantillon (1, 1a) avec une chambre d'échantillon (2) pour un échantillon (3) se formant à partir d'une masse fondue présentant au moins un dissipateur thermique inférieur (6), au moins un dissipateur thermique supérieur (8) et au moins un dissipateur thermique interne (7) et au moins une pièce de remplissage, dans lequel la chambre d'échantillon (2) est entourée au moins par le dissipateur thermique inférieur (6) et par le dissipateur thermique interne (7) ensemble de sorte qu'au moins la chambre d'échantillon (2) peut être refroidie au moins au moyen du dissipateur thermique inférieur et interne (6, 7), dans lequel la pièce de remplissage connectée à la chambre d'échantillon (2) débouche avec une ouverture de remplissage (5a) dans la chambre d'échantillon (2) et dans lequel les dissipateurs thermiques (6, 7, 8) présentent chacun une face externe (7a, 8a), **caractérisé en ce que** le dispositif de prélèvement d'échantillon (1, 1a) présente entre une région de la face externe (7a) du dissipateur thermique interne (7) et la région de la face externe (8a) du dissipateur thermique supérieur (8) opposée à cette région de la face externe (7a) du dissipateur thermique interne (7) au moins une fente (11) pour le transport d'au moins un gaz et que le volume du dissipateur thermique respectif (6, 7, 8) est supérieur au volume de la fente (11), qu'au moins un des dissipateurs thermiques (6, 7, 8) présente au moins une ouverture d'aération (16) et que l'ouverture d'aération (16) peut être fermée avec au moins une fermeture ouvrable, de préférence une membrane ouvrable (17), dans lequel la fermeture s'ouvre pendant ou après le remplissage de la masse fondue dans la chambre d'échantillon (2).

2. Dispositif de prélèvement d'échantillon (1, 1a) selon la revendication 1, **caractérisé en ce qu'**au moins le dissipateur thermique inférieur (6) et le dissipateur thermique interne (7) forment une paroi de la chambre d'échantillon (2), dans lequel la paroi est formée par une région de la face externe respective du dissipateur thermique inférieur (6) et du dissipateur thermique interne (7) de sorte qu'une chambre d'échantillon (2) avec un espace creux se forme entre le dissipateur thermique inférieur (6) et le dissipateur thermique interne (7).

3. Dispositif de prélèvement d'échantillon (1, 1a) selon la revendication 1 ou la revendication 2, **caractérisé en ce que** l'ouverture d'aération (16) présente un diamètre d'environ 0,7 mm à environ 1,3 mm.

4. Dispositif de prélèvement d'échantillon (1, 1a) selon une des revendications 1 ou 3, **caractérisé en ce que** la fermeture, de préférence la membrane (17), présente au moins une connexion plastique.

5. Dispositif de prélèvement d'échantillon (1, 1a) selon une des revendications 1 à 4, **caractérisé en ce que** la fermeture présente une résistance à la pression d'environ 0,5 bar à environ 4 bars.

6. Dispositif de prélèvement d'échantillon (1, 1a) selon une des revendications 1 à 5, **caractérisé en ce que** la fermeture présente une résistance à la température d'environ 50°C à environ 90°C.

7. Procédé de prélèvement d'échantillon d'une masse fondue avec une température de fusion supérieure à 600°C, dans lequel le dispositif de prélèvement d'échantillon (1, 1a) positionné à une extrémité d'une lance et/ou d'une pièce de support selon une des revendications 1 à 6 est immergé dans la masse fondue, dans lequel la chambre d'échantillon (2) du dispositif de prélèvement d'échantillon (1, 1a) est ensuite remplie de masse fondue et dans lequel au moins l'échantillon (3) est ensuite extrait de la masse fondue à l'aide du dispositif de prélèvement d'échantillon (1, 1a), **caractérisé en ce qu'**au moins un gaz est amené dans le dispositif de prélèvement d'échantillon (1, 1a) avant l'immersion, dans lequel le gaz s'écoule de nouveau du dispositif de prélèvement d'échantillon (1, 1a) à travers au moins une pièce de remplissage, le dispositif de prélèvement d'échantillon (1, 1a) est ensuite immergé dans la masse fondue, l'amenée de gaz est ensuite modifiée, la chambre d'échantillon (2) se remplit ensuite de masse fondue, du gaz est ensuite de nouveau amené dans le dispositif de prélèvement d'échantillon (1, 1a) pendant ou après le remplissage de la chambre d'échantillon (2) de masse fondue de sorte qu'au moins la chambre d'échantillon (2) est refroidie par le gaz amené, que le gaz s'écoulant de l'ouverture d'aération (16) est évacué à travers au moins une ouverture de sortie de gaz (18) pour la sortie du gaz amené du dispositif de prélèvement d'échantillon (1, 1a) et que la fermeture est perméable au gaz pendant ou après le remplissage de la masse fondue dans la chambre d'échantillon (2) au moyen de la température de la masse fondue et/ou de la pression du gaz amené.

8. Procédé selon la revendication 7, **caractérisé en ce que** le gaz s'écoule à travers au moins une fente (11) entre au moins le dissipateur thermique interne (7) et le dissipateur thermique supérieur (8).

9. Procédé selon la revendication 7 ou 8, **caractérisé en ce que** le gaz ne s'écoule que de la pièce de remplissage avant le versement de la masse fondue et s'écoule à travers au moins une ouverture d'aération (16) pendant ou après le remplissage de la masse fondue dans la chambre d'échantillon (2).

10. Procédé selon une des revendications précédentes, **caractérisé en ce que** le gaz de nouveau amené s'écoule à travers la fente (11) après le remplissage de la chambre d'échantillon (2) de masse fondue et le gaz s'écoule ensuite de nouveau du dispositif de prélèvement d'échantillon (1, 1a) à travers l'ouverture d'aération (16), dans lequel la température de l'échantillon (3) est de ce fait abaissée par refroidissement.

11. Procédé selon une des revendications précédentes, **caractérisé en ce que** l'échantillon (3) est maintenu au moyen du dissipateur thermique inférieur (6) et/ou que le dissipateur thermique interne (7) est maintenu au moyen du dissipateur thermique supérieur (8).

12. Procédé selon une des revendications précédentes, **caractérisé en ce que** l'échantillon (3) est amené dans le dispositif de prélèvement d'échantillon (1, 1a) d'une installation d'analyse.
